# EUROPEAN PATENT SPECIFICATION

(11) **EP 4 447 957 B1**
(45) Date of publication and mention of the grant of the patent: **17.09.2025**
(21) Application number: 22839652.9
(22) Date of filing: 15.12.2022
(51) Int. Cl.: A61K 31/353, A61P 25/28

(54) **COMPOSITIONS FOR THE TREATMENT OF FGFR3-RELATED COGNITIVE DEFICITS WITH A CATECHIN**
ZUSAMMENSETZUNGEN ZUR BEHANDLUNG VON FGFR3-VERWANDTEN KOGNITIVEN DEFIZITEN MIT EINEM CATECHIN
COMPOSITIONS DESTINÉS AU TRAITEMENT DE DÉFICITS COGNITIFS LIÉS À FGFR3 AVEC UNE CATÉCHINE

(30) Priority: 16.12.2021 EP 21306791
(43) Date of publication of application: 23.10.2024
(73) Proprietor: Institut National de la Santé et de la Recherche Médicale (INSERM), 75013 Paris (FR); Université Paris Cité, 75006 Paris (FR); Fondation Imagine, 75015 Paris (FR); APHP (Assistance Publique - Hôpitaux de Paris), 75012 Paris (FR)
(72) Inventor: LEGEAI-MALLET, Laurence, 75015 Paris (FR)
(74) Representative: Schulz Junghans Patentanwälte PartGmbB
(86) International application number: PCT/EP2022/086107
(87) International publication number: WO 2023/111165

(56) References cited:
- WO-A1-2019/145356
- KACI NABIL ET AL: "Theobroma cacao extracts as a treatment for FGFR3-related disorders", CALCIFIED TISSUE INTERNATIONAL, vol. 104, no. Suppl 1, 19 April 2019 (2019-04-19), pages S37, XP055920039, DOI: 10.1007/s00223-019-00544-x
- "Gene Reviews", vol. 15, 7 May 2020 (2020-05-07), pages 1 - 22, XP055919817, Retrieved from the Internet <URL:https://www.ncbi.nlm.nih.gov/books/NBK1477/pdf/Bookshelf_NBK1477.pdf>
- ARNAUD-LÓPEZ L ET AL: "Crouzon with acanthosis nigricans. Further delineation of the syndrome", CLINICAL GENETICS, WILEY-BLACKWELL MUNKSGAARD, DK, vol. 72, no. 5, 2 September 2007 (2007-09-02), pages 405 - 410, XP071080694, ISSN: 0009-9163, DOI: 10.1111/J.1399-0004.2007.00884.X
- VAN PRAAG H. ET AL: "Plant-Derived Flavanol (-)Epicatechin Enhances Angiogenesis and Retention of Spatial Memory in Mice", THE JOURNAL OF NEUROSCIENCE, vol. 27, no. 22, 30 May 2007 (2007-05-30), US, pages 5869 - 5878, XP055920150, ISSN: 0270-6474, DOI: 10.1523/JNEUROSCI.0914-07.2007
- HASKELL-RAMSAY CRYSTAL ET AL: "The Impact of Epicatechin on Human Cognition: The Role of Cerebral Blood Flow", NUTRIENTS, vol. 10, no. 8, 27 July 2018 (2018-07-27), pages 986, XP055920146, DOI: 10.3390/nu10080986

## Description

### FIELD OF THE INVENTION:

The present invention is in the field of medicine, in particular skeletal diseases and neurology.

### BACKGROUND OF THE INVENTION:

The Fibroblast Growth Factor Receptor (FGFR) family plays an important role in bone development and skeletal diseases. FGFR1, FGFR2 and FGFR3 missense mutations are responsible for a spectrum of syndromic craniosynostoses characterized by premature fusion of cranial sutures (Robin et al., 1993; Twigg and Wilkie, 2015). Two specific FGFR3 dominant mutations account for Crouzon syndrome with acanthosis nigricans (CAN [MIM 612247]), a rare syndromic craniosynostosis, and Muenke syndrome (MS [MIM 602849]), the most common syndromic craniosynostosis (Wilkie et al., 2010). CAN patients present with acanthosis nigricans but otherwise resemble to FGFR2-related Crouzon syndrome patients: they are characterized by the premature fusion of the coronal sutures of the skull, brachycephaly, midface hypoplasia and cranio-vertebral junction anomalies (Arnaud-López et al., 2007; Di Rocco et al., 2011; Meyers et al., 1995; Mir A et al., 2013. CAN is defined by a single point mutation (p.Ala391Glu) localized in transmembrane domain of FGFR3 (Li et al., 2006; Meyers et al., 1995). The potential consequences of abnormal skull vault, skull base and facial growth include increased intracranial pressure, hearing and vision impairments, impaired brain blood flow, hindbrain malformation, syringomyelia, sleep apnea and multifactorial developmental delay. Several studies have reported cognitive deficit in FGFR3-related craniosynostoses characterized by impairment of memory capacity, attention, anxiety, and emotional control (Kruszka et al., 1993; Maliepaard et al., 2014; Yarnell et al., 2015). FGFR3 is widely recognized as an important regulator of the endochondral ossification. However, its role in sutural growth biology and membranous ossification are less known. The role of the p.Ala391Glu CAN mutation remains unexplored. The inventors generated the first CAN mouse model (*Fgfr3*^{*A385E*/*+*}) expressing a dominant p.Ala385Glu mutation. The *Fgfr3*^{*A385E*/*+*} mice showed an absence of craniosynostosis and normal craniocerebral proportion. In the central nervous system, FGFR3 is highly expressed in the hippocampus, brain structure essential for cognition mechanisms.

FGFR3-related chondrodysplasia such as achondroplasia, Severe Achondroplasia with developmental delay and acanthosis nigricans (SADDAN), and hypochondroplasia (HCH) are characterized by short limbs, skull base anomalies, macrocephaly, deafness, hypoplasia of the midface, prognathism and less common but significant clinical features, Mild-to-moderate intellectual disability and learning disability . The inventors generated the first HCH mouse model (*Fgfr3*^{*N534Lys*/*+*}) expressing a dominant p.Asn540Lys mutation. The *Fgfr3*^{*A385E*/*+*} mice showed a mild dwarfism, an absence of craniosynostosis and normal craniocerebral proportion and a defective memory.

However the impact of a FGFR3 gain of function mutation in cognitive behaviors has never been investigated.

### SUMMARY OF THE INVENTION:

The present invention is defined by the claims. In particular, the present invention relates to methods and compositions for the treatment of a cognitive deficit that is caused by an abnormal overactivation of FGFR3 in the brain.

The references to the methods of treatment of this description are to be interpreted as references to compounds, pharmaceutical compositions and medicaments of the present invention for use in a method for treatment of the human (or animal) body by therapy.

### DETAILED DESCRIPTION OF THE INVENTION:

Fibroblast Growth Factor Receptor 3 (FGFR3) gain-of-function mutations (p.Ala391Glu) is responsible for a rare form of craniosynostosis: Crouzon syndrome with Acanthosis Nigricans (CAN). The patients with CAN are characterized by premature fusion of coronal sutures of the skull, midface hypoplasia, acanthosis nigricans and neurological impairment. FGFR3 is defined as a negative regulator of long bone growth. However, the role of the p.Ala391Glu CAN mutation in sutural growth biology of the skull remains unexplored. The inventors observed that the CAN mutation induced an overactivation of receptor independently of ligand and disturbed the protein maturation. The inventors generated the first CAN mouse model (*Fgfr3*^{*A385E*/*+*}) expressing a dominant p.Ala385Glu mutation and a HCH mouse model (*Fgfr3*^{*N534Lys*/*+*}) expressing a dominant p.Asn540Lys mutation. The *Fgfr3*^{*A385E*/*+*} mice showed an absence of craniosynostosis and normal craniocerebral proportion. However, analyzing adult hippocampus of these mice CAN and HCH, the inventors showed that FGFR3 overactivation was associated to decrease dentate gyrus progenitor proliferation and neurogenesis. Consequently, behavioral tests were performed in *Fgfr3*^{*A385E*/*+*} mice and hippocampal-dependent memory impairment and abnormal coping strategy were observed. Lastly, using a catechin, the inventors inhibited the FGFR3 overactivation in the brain of *Fgfr3*^{*A385E*/*+*} mice and *Fgfr3*^{*A385E*/*+*} mice thus restoring the behavioral and cognitive defects. This highlights for the first-time behavior anomalies associated to Fgfr3 overactivation in the brain. It allows a better understanding of the role played by FGFR3 in learning processes and emotional responses in craniosynostoses and chondrodysplasia.

Accordingly, the first object of the present invention relates to a method of treating a cognitive deficit that is caused by an abnormal overactivation of FGFR3 in the brain in a subject in need thereof comprising administering to the subject a therapeutically effective amount of at least one catechin.

As used herein, the term **"subject"** or **"patient** is suffering or will suffer from a FGFR3-related cognitive deficit. In some embodiments, the patient is a child. In some embodiments, the patient is a premature infant. In some embodiments, the patient is less than 15 years old. In some embodiments, the patient is less than 10 years old. In some embodiments, the patient is less than 7 years old. In some embodiments, the patient is less than 5 years old. In some embodiments, the patient is less than 3 years old. In some embodiments, the patient is an adult. In some embodiments, the subject is more than 15 years old. In some embodiments, the subject is more than 20 years old. In some embodiments, the subject is more than 25 years old. In some embodiments, the subject is more than 30 years old. In some embodiments, the subject is more than 35 years old. In some embodiments, the patient is an elderly.

In some embodiment, the subject harbours a constitutively active FGFR3 receptor variant.

In some embodiment, the subject harbours a FGFR3 gain-of-function mutation.

As used herein, the term **"Fibroblast growth factors"** (FGF) relates a family of cell signalling proteins produced by macrophages; they are involved in a wide variety of processes, most notably as crucial elements for normal development in animal cells. Any irregularities in their function lead to a range of developmental defects. These growth factors typically act as systemic or locally circulating molecules of extracellular origin that activate cell surface receptors. A defining property of FGFs is that they bind to heparin and to heparan sulfate. Thus, some are sequestered in the extracellular matrix of tissues that contains heparan sulfate proteoglycans and are released locally upon injury or tissue remodeling.

As used herein, the term **"Fibroblast growth factor receptors"** (FGFR) relates receptors that bind to members of the fibroblast growth factor (FGF) family of proteins. Some of these receptors are involved in pathological conditions. Distinct membrane FGFR have been identified in vertebrates and all of them belong to the tyrosine kinase superfamily: FGFR1 (see also Fibroblast growth factor receptor 1) (= CD331), FGFR2 (see also Fibroblast growth factor receptor 2) (= CD332), FGFR3 (see also Fibroblast growth factor receptor 3) (= CD333), FGFR4 (see also Fibroblast growth factor receptor 4) (= CD334), FGFRL1 (see also Fibroblast growth factor receptor-like 1) and FGFR6.

As used herein, the terms "FGFR3", **"FGFR3 tyrosine kinase receptor"** and **"FGFR3 receptor"** are used interchangeably throughout the specification and refer to all of the naturally-occurring isoforms of FGFR3. An exemplary human amino acid sequence of FGFR3 is represented by SEQ ID NO:1.

SEQ ID NO:1 >sp|P22607|FGFR3_HUMAN Fibroblast growth factor receptor
3 OS=Homo sapiens OX=9606 GN=FGFR3 PE=1 SV=1
MGAPACALALCVAVAIVAGASSESLGTEQRVVGRAAEVPGPEPGQQEQLVFGSGDAVELS
CPPPGGGPMGPTVWVKDGTGLVPSERVLVGPQRLQVLNASHEDSGAYSCRQRLTQRVLCH
FSVRVTDAPSSGDDEDGEDEAEDTGVDTGAPYWTRPERMDKKLLAVPAANTVRFRCPAAG
NPTPSISWLKNGREFRGEHRIGGIKLRHQQWSLVMESVVPSDRGNYTCVVENKFGSIRQT
YTLDVLERSPHRPILQAGLPANQTAVLGSDVEFHCKVYSDAQPHIQWLKHVEVNGSKVGP
DGTPYVTVLKTAGANTTDKELEVLSLHNVTFEDAGEYTCLAGNSIGFSHHSAWLVVLPAE
EELVEADEAGSVYAGILSYGVGFFLFILVVAAVTLCRLRSPPKKGLGSPTVHKISRFPLK
RQVSLESNASMSSNTPLVRIARLSSGEGPTLANVSELELPADPKWELSRARLTLGKPLGE
GCFGQVVMAEAIGIDKDRAAKPVTVAVKMLKDDATDKDLSDLVSEMEMMKMIGKHKNIIN
LLGACTQGGPLYVLVEYAAKGNLREFLRARRPPGLDYSFDTCKPPEEQLTFKDLVSCAYQ
VARGMEYLASQKCIHRDLAARNVLVTEDNVMKIADFGLARDVHNLDYYKKTTNGRLPVKW
MAPEALFDRVYTHQSDVWSFGVLLWEIFTLGGSPYPGIPVEELFKLLKEGHRMDKPANCT
HDLYMIMRECWHAAPSQRPTFKQLVEDLDRVLTVTSTDEYLDLSAPFEQYSPGGQDTPSS
SSSGDDSVFAHDLLPPAPPSSGGSRT

As used herein, the expressions **"FGFR3 gain-of-function mutation", "constitutively active FGFR3 receptor variant", "constitutively active mutant of the FGFR3"** or **"mutant FGFR3 displaying a constitutive activity"** are used interchangeably and refer to a mutant of said receptor exhibiting a biological activity (*i.e.* triggering downstream signaling), and/or exhibiting a biological activity which is higher than the biological activity of the corresponding wild-type receptor in the presence of FGF ligand. A constitutively active FGFR3 variant according to the invention is in particular chosen from the group consisting of (residues are numbered according to their position in the precursor of fibroblast growth factor receptor 3 isoform 1 - 806 amino acids long -): a mutant wherein the serine residue at position 84 is substituted with lysine (named herein below S84L); a mutant wherein the arginine residue at position 200 is substituted with cysteine (named herein below R200C); a mutant wherein the arginine residue at position 248 is substituted with cysteine (named herein below R248C); a mutant wherein the serine residue at position 249 is substituted with cysteine (named herein below S249C); a mutant wherein the proline residue at position 250 is substituted with arginine (named herein below P250R); a mutant wherein the asparagine residue at position 262 is substituted with histidine (named herein below N262H); a mutant wherein the glycine residue at position 268 is substituted with cysteine (named herein below G268C); a mutant wherein the tyrosine residue at position 278 is substituted with cysteine (named herein below Y278C); a mutant wherein the serine residue at position 279 is substituted with cysteine (named herein below S279C); a mutant wherein the glycine residue at position 370 is substituted with cysteine (named herein below G370C); a mutant wherein the serine residue at position 371 is substituted with cysteine (named herein below S371C); a mutant wherein the tyrosine residue at position 373 is substituted with cysteine (named herein below Y373C); a mutant wherein the glycine residue at position 380 is substituted with arginine (named herein below G380R); a mutant wherein the valine residue at position 381 is substituted with glutamate (named herein below V381E); a mutant wherein the alanine residue at position 391 is substituted with glutamate (named herein below A391E); a mutant wherein the asparagine residue at position 540 is substituted with Lysine (named herein below N540K); a mutant wherein the termination codon is eliminated due to base substitutions, in particular the mutant wherein the termination codon is mutated in an arginine, cysteine, glycine, serine or tryptophane codon (named herein below X807R, X807C, X807G, X807S and X807W, respectively); a mutant wherein the lysine residue at position 650 is substituted with another residue, in particular with methionine, glutamate, asparagine or glutamine (named herein below K650M, K650E, K650N and K650Q); a mutant wherein the methionine residue at position 528 is substituted with isoleucine (named herein below M528I); a mutant wherein the isoleucine residue at position 538 is substituted with valine (named herein below I538V); a mutant wherein the asparagine residue at position 540 is substituted with serine (named herein below N540S); a mutant wherein the asparagine residue at position 540 is substituted with threonine (named herein below N540T). Typically, a constitutively active FGFR3 variant according to the invention is N540K, K650N, K650Q, M528I, I538V, N540S, N540T or A391E mutant.

As used herein, the term **"cognitive deficit"** relates to a set of symptoms including depression, memory, perception, slowness, and difficulty solving problems. Cognitive deficit may exist as symptoms in some mental disorders (psychoses, mood disorders, anxiety disorders), but they are primarily synonymous with brain damage. As used herein, the term **"FGFR3-related cognitive deficit"** is intended to mean a cognitive deficit that is caused by an abnormal over-activation of FGFR3 in the brain, in particular by expression of a constitutively active mutant of the FGFR3receptor, in particular a constitutively active mutant of the FGFR3 receptor as described above.

In some embodiment, the subject has or will have a cognitive and behavior deficit selected from the group consisting of episodic memory deficit, antidepressant effect, anomalies in learning and stress response.

In particular, the constitutively active FGFR3 receptor variant induces brain morphological abnormalities, learning and memory deficit, and/or a decrease of neurogenesis. As used herein, the term **"neurogenesis"** has its general meaning in the art and relates to the process by which new neurons are formed in the brain. Neurogenesis is crucial when an embryo is developing, but also continues in certain brain regions after birth and throughout our lifespan. The mature brain has many specialized areas of function, and neurons that differ in structure and connections. The hippocampus, for example, which is a brain region that plays an important role in memory and spatial navigation, alone has at least 27 different types of neurons. The incredible diversity of neurons in the brain results from regulated neurogenesis during embryonic development. During the process, neural stem cells differentiate-that is, they become any one of a number of specialized cell types-at specific times and regions in the brain.

In some embodiment, the subject suffers from a skeletal disease that is caused by an abnormal increased activation of FGFR3.

As used herein the term **"FGFR3-related skeletal disease"** is intended to mean a skeletal disease that is caused by an abnormal increased activation of FGFR3, in particular by expression of a constitutively active mutant of the FGFR3 receptor, in particular a constitutively active mutant of the FGFR3 receptor as described above.

In some embodiment, the FGFR3-related skeletal diseases are preferably FGFR3-related chondrodysplasias and FGFR3-related craniosynostosis.

As used herein **"FGFR3-related chondrodysplasias"** include but are not limited to dwarfism such as hypochondroplasia (HCH), thanatophoric dysplasia (TD) type I, thanatophoric dysplasia type II, achondroplasia (ACH) and SADDAN (severe achondroplasia with developmental delay and acanthosis nigricans).

In particular, the FGFR3-related skeletal disease is dwarfism.

As used herein, the term **"dwarfism"** has its general meaning in the art and refers to a short stature that results from a genetic or medical condition. Dwarfism is generally defined as an adult height of 147 centimeters or less.

In particular, the FGFR3-related skeletal disease is hypochondroplasia (HCH).

As used herein, the term **"hypochondroplasia"** (HCH) has its general meaning in the art and relates to a disproportionately short stature, rhizomelia and a head that appears large in comparison with the underdeveloped portions of the body and mild-to-moderate intellectual disability.

In some embodiment, the FGFR3-related chondrodysplasias is a hypochondroplasia caused by expression of the N540K, K650N, K650Q, M528I, 1538V, N540S or N540T constitutively active mutant of the FGFR3 receptor.

In particular, the FGFR3-related skeletal disease is achondroplasia (ACH).

As used herein, the term **"achondroplasia"** (ACH) has its general meaning in the art and relates to a genetic deficit in which the arms and legs are short, while the torso is typically of normal length and with an enlarged head and prominent forehead.

In particular, the FGFR3-related skeletal disease is thanatophoric dysplasia (TD).

As used herein, the term **"thanatophoric dysplasia"** (TD) has its general meaning in the art and relates to a severe skeletal deficit characterized by a disproportionately small ribcage, extremely short limbs and folds of extra skin on the arms and legs.

In some embodiment, the FGFR3-related skeletal disease is FGFR3-related craniosynostosis. In some embodiments, the FGFR3-related craniosynostosis corresponds to an inherited or to a sporadic disease.

In particular, the FGFR3-related craniosynostosis is Muenke syndrome caused by expression of the P250R constitutively active mutant of the FGFR3 receptor.

In particular, the FGFR3-related craniosynostosis is Crouzon syndrome with acanthosis nigricans (CAN) caused by expression of the A391E constitutively active mutant of the FGFR3 receptor.

As used herein, the term **"craniosynostosis"** has its general meaning in the art and relates a condition in which one or more of the fibrous sutures a patient skull prematurely fuses by turning into bone (ossification), thereby changing the growth pattern of the skull. "Crouzon syndrome with acanthosis nigricans" (CAN) is a very rare craniosynostosis.

As used herein, the term **"acanthosis nigricans"** relates to a brown to black, poorly defined, velvety hyperpigmentation of the skin.

As used herein, the term **"treatment"** or **"treat"** refer to both prophylactic or preventive treatment as well as curative, improving the patient's condition or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical deficit or who ultimately may acquire the deficit, in order to prevent, cure, delay the onset of, reduce the severity of, or ameliorate one or more symptoms of a deficit or recurring deficit, or in order to prolong the survival of a patient beyond that expected in the absence of such treatment. By **"therapeutic regimen"** is meant the pattern of treatment of an illness, e.g., the pattern of dosing used during therapy. A therapeutic regimen may include an induction regimen and a maintenance regimen. The phrase **"induction regimen"** or **"induction period"** refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) **a "loading regimen",** which may include administering a greater dose of the drug than a physician would employ during a maintenance regimen, administering a drug more frequently than a physician would administer the drug during a maintenance regimen, or both. The phrase **"maintenance regimen"** or **"maintenance period"** refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at regular intervals, e.g., daily, weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term **"preventing"** intends characterizing a prophylactic method or process that is aimed at delaying or preventing the onset of a deficit or condition to which such term applies.

The term **"expression"** when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein (i.e. FGFR3) produced by translation of a mRNA.

As used herein, the term **"catechin"** is a flavan-3-ol, a type of natural phenol and antioxidant having the general formula (I):

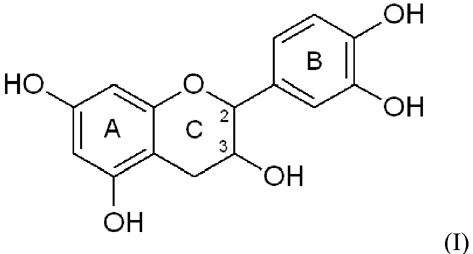

Catechins are plant secondary metabolites and belong to the group of flavan-3-ols (or simply flavanols), part of the chemical family of flavonoids. Catechin possesses two benzene rings (called the A- and B-rings) and a dihydropyran heterocycle (the C-ring) with a hydroxyl group on carbon 3. The A ring is similar to a resorcinol moiety while the B ring is similar to a catechol moiety. There are two chiral centers on the molecule on carbons 2 and 3. Therefore, catechin has four diastereoisomers: two of the isomers are in trans configuration and are called catechin and the other two are in cis configuration and are called epicatechin. The most common catechin isomer is the (+)-catechin. The other stereoisomer is (-)-catechin or ent-catechin. The most common epicatechin isomer is (-)-epicatechin (also known under the names L-epicatechin, epicatechol, (-)-epicatechol, l-acacatechin, l-epicatechol, epi-catechin, 2,3-cis-epicatechin or (2R,3R)-(-)-epicatechin). Methods of producing, synthetizing or extracting catechins are well known to the skilled person.

In some embodiment, the catechin isomer is (+)-catechin (2R,3S). As used herein the term "(+)-**catechin"** has its general meaning in the art and has the following formula:

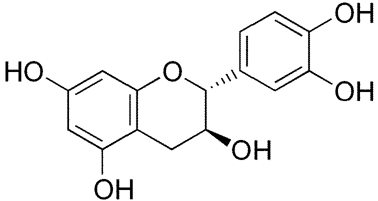

In some embodiment, the catechin isomer is (-)-catechin (2S,3R). As used herein the term "(-)-catechin" has its general meaning in the art and has the following formula:

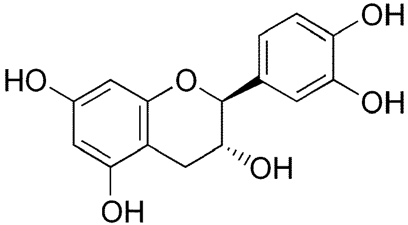

In some embodiment, the epicatechin isomer is (+)-epicatechin (2S,3 S). As used herein the term "(+)-epicatechin" has its general meaning in the art and is having the following formula:

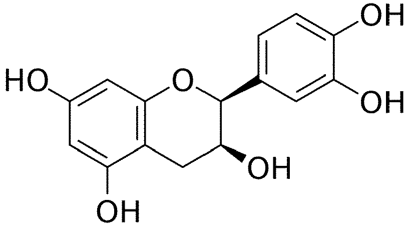

In some embodiment, the epicatechin isomer is (-)-epicatechin (2R,3R). As used herein the term **"(-)-epicatechin"** has its general meaning in the art and refers to (2R,3R)-2-(3,4-dihydroxyphenyl)-3,4-dihydro-2H-chromene-3,5,7-triol and is having the following formula:

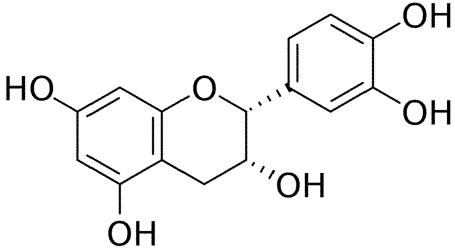

In some embodiment, the (-)-epicatechin is a substantially pure (-)-epicatechin. As used herein, the term **"substantially pure"** refers to the total absence, or near total absence, of impurities, such as related-substance impurities. For example, when a (-)-epicatechin composition is said to be substantially pure, there are either no detectable related-substance impurities, or if a single related-substance impurity is detected, it is present in an amount no greater than 0.1 % by weight, or if multiple related- substance impurities are detected, they are present in aggregate in an amount no greater than 0.6% by weight. The substantially pure (-)-epicatechin according to the invention are capable of inhibiting or eliminating the functional activation of the FGFR3 downstream signalling pathway in vivo and/or in vitro. The a substantially pure (-)-epicatechin may inhibit the functional activation of FGFR3 downstream signalling pathway by at least about 10%, preferably by at least about 20%, preferably by at least about 30%, preferably by at least about 40%, preferably by at least about 50%, preferably by at least about 70, 75 or 80%, still preferably by 85, 90, 95, or 100% or preferably by at least about 300% or preferably by at least about 500%.

In some embodiments, the subject is administered with a pharmaceutical composition comprising the therapeutically effective amount of catechin.

As used herein the terms **"administering"** or **"administration"** refer to the act of delivering a substance as it exists outside the body (e.g. a catechin) into the patient, such as by oral delivery (e.g bottle treatment) , intracerebroventricular, mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

According to the invention, the catechin is administered to the patient using any suitable method that enables the catechin to reach the brain. In some embodiments, the catechin administered to the patient orally (i.e. bottle treatment) or systemically (i.e. via systemic administration). Thus, in some embodiments, the catechin is administered to the patient such that it enters the circulatory system and is distributed throughout the body. In some embodiments, the catechin is administered to the patient by local administration, for example by local administration to the hypothalamus.

A **"therapeutically effective amount"** refers to an amount effective, at dosages and for periods of time necessary, to achieve a desired therapeutic result. A therapeutically effective amount of catechin may vary according to factors such as the disease state, age, sex, and weight of the individual, and the ability of the catechin to elicit a desired response in the individual. A therapeutically effective amount is also one in which any toxic or detrimental effects of the catechin are outweighed by the therapeutically beneficial effects. The efficient dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. One of ordinary skill in the art would be able to determine such amounts based on such factors as the patient's size, the severity of the patient's symptoms, and the particular composition or route of administration selected. An exemplary, non-limiting range for a therapeutically effective amount of the catechin is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some embodiments, the efficacy of the treatment is monitored during the therapy, e.g. at predefined points in time. As non-limiting examples, treatment according to the present invention may be provided as a daily dosage of the agent of the present invention in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

In some embodiments, the patient is administered with a pharmaceutical composition comprising the therapeutically effective amount of catechin as active principle and at least one pharmaceutically acceptable excipient.

As used herein the term **"active principle"** or **"active ingredient"** are used interchangeably. As used herein, the term **"pharmaceutical composition"** refers to a composition described herein, or pharmaceutically acceptable salts thereof, with other agents such as carriers and/or excipients. The pharmaceutical compositions as provided herewith typically include a pharmaceutically acceptable carrier.

As used herein, the term **"pharmaceutically acceptable carrier"** includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical-Sciences, Sixteenth Edition, E. W. Martin (Mack Publishing Co., Easton, Pa., 1980) discloses various carriers used in formulating pharmaceutical compositions and known techniques for the preparation thereof.

A pharmaceutically acceptable carrier or excipient refers to a non-toxic solid, semi-solid or liquid filler, diluent, encapsulating material or formulation auxiliary of any type. Typically, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Sterile injectable solutions are prepared by incorporating the agent of the present invention in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In some embodiments, the subject is administered with a food composition comprising the therapeutically effective amount of the catechin.

Typically, the food composition comprises or consists of a food product, a food additive, a food ingredient, a functional food, a medical food, a dietary supplement, a nutraceutical, a nutritional supplement or an oral preparation comprising an effective amount of catechin. In some embodiments, the food composition is elected from complete food compositions, food supplements, nutraceutical compositions, and the like. The composition of the present invention may be used as a food ingredient and/or feed ingredient. The food ingredient may be in the form of a solution or as a solid-depending on the use and/or the mode of application and/or the mode of administration. The food composition of the present invention may be made using known methods, such as adding an effective amount of the catechin to a food base. The composition that comprises the food product, the food additive, the food ingredient, the functional food, the medical food, the dietary supplement, the nutraceutical, the nutritional supplement or the oral preparation of the present invention may be solid, semi-solid or liquid.

It may be in the form of a food product or food supplement, e.g. in the form of tablets, gels, powders, capsules, drinks, bars, bottle treatment etc. For example the composition may be in the form of a powder packed in a sachet which can be dissolved in water, fruit juice, milk or another beverage.

As used herein, the term **"food"** refers to liquid (i.e. drink), solid or semi-solid dietetic compositions, especially total food compositions (food-replacement), which do not require additional nutrient intake or food supplement compositions. Food supplement compositions do not completely replace nutrient intake by other means. As used herein the term the **"food product",** the **"food additive",** the **"food ingredient"** or the **"feed ingredient"** includes a formulation which is or can be added to functional foods or foodstuffs as a nutritional supplement. By **"nutritional food"** or **"nutraceutical"** or **"functional food",** is meant a foodstuff which contains ingredients having beneficial effects for health or capable of improving physiological functions. By **"food supplement",** is meant a foodstuff having the purpose of completing normal food diet. A food supplement is a concentrated source of nutrients or other substances having a nutritional or physiological effect, when they are taken alone or as a combination in small amounts. As used herein the term **"functional food"** summarizes foodstuff and corresponding products lately developed to which importance is attributed not only due to them being valuable as to nutrition and taste but due to particular ingredient substances. According to the invention, the middle- or long-term maintenance and promotion of health are of importance. In this context, non-therapeutic uses are preferred. The preventive aspect and the promotion of health as well as the food character of the products are, however, best made clear by the term functional food. In many cases, these relate to products accumulated by assortment and selection (as is also the case in the present invention), purification, concentration, increasingly also by addition. Isolated effective substances, in particular in form of tablets or pills, are not included. Although there is no legal definition of a functional food, most of the parties with an interest in this area agree that they are foods marketed as having specific health effects beyond basic nutritional effects. Accordingly, functional foods are ordinary foods that have components or ingredients (such as those described herein) incorporated into them that impart to the food a specific functional e.g. medical or physiological benefit other than a purely nutritional effect.

In some embodiments, the food composition is a drink is a functional drink or a therapeutic drink, a thirst-quencher or an ordinary drink. By way of example, the composition of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.

The food composition that comprises the food product, the food additive, the food ingredient, the functional food, the medical food, the dietary supplement, the nutraceutical, the nutritional supplement or the oral preparation of the present invention typically comprises carriers or vehicles. **"Carriers"** or **"vehicles"** mean materials suitable for administration and include any such material known in the art such as, for example, any liquid, gel, solvent, liquid diluent, solubilizer, or the like, which is non-toxic and which does not interact with any components of the composition in a deleterious manner. Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like. In some embodiments, the composition that comprises the food product, the food additive, the food ingredient, the functional food, the medical food, the dietary supplement, the nutraceutical, the nutritional supplement or the oral preparation of the present invention contains protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The composition may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

The invention will be further illustrated by the following figures and examples. However, these examples and figures should not be interpreted in any way as limiting the scope of the present invention.

### FIGURES:

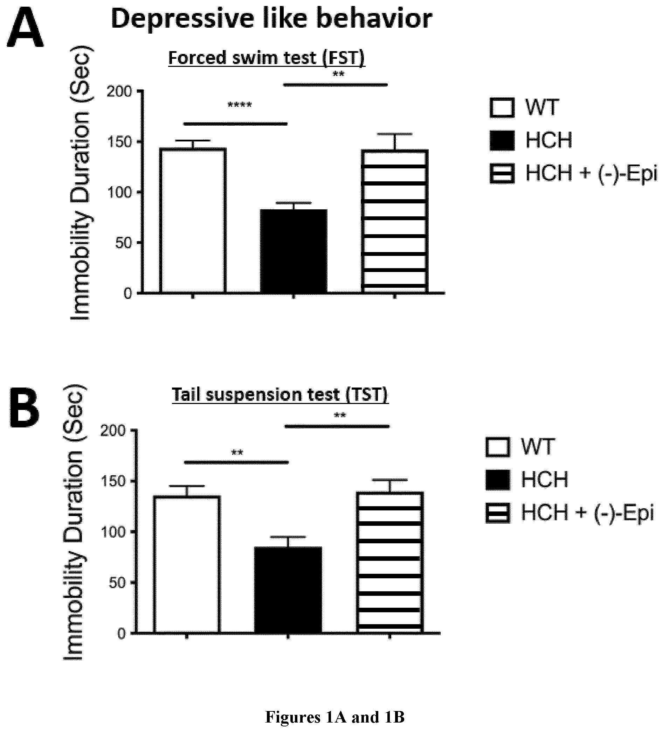

**Figure 1****: Learning impairment and antidepressant-like behavior in Hypochondroplasia (HCH) mouse model.** A. Forced swim test (FST) performed in 4 month-old male animals in two independent groups. FST was performed during two consecutive days. Immobility duration was assessed for both day 1 and day 2. (*Fgfr3*^{*+*/}*⁺,* n=15; *Fgfr3*^{*N534K*/*+,*} n=9, *Fgfr3*^{*N534K*/}*⁺ +* (-)-Epi, n=10). **B.** Tail suspension test (TST) performed in 4 month-old male animals during two consecutive days and immobility duration was assessed for both (*Fgfr3*^{*+*/}*⁺,* n=15; *Fgfr3*^{*N534K*/*+,*} n=9, *Fgfr3*^{*N534K*/*+*} + (-)-Epi, n=10).

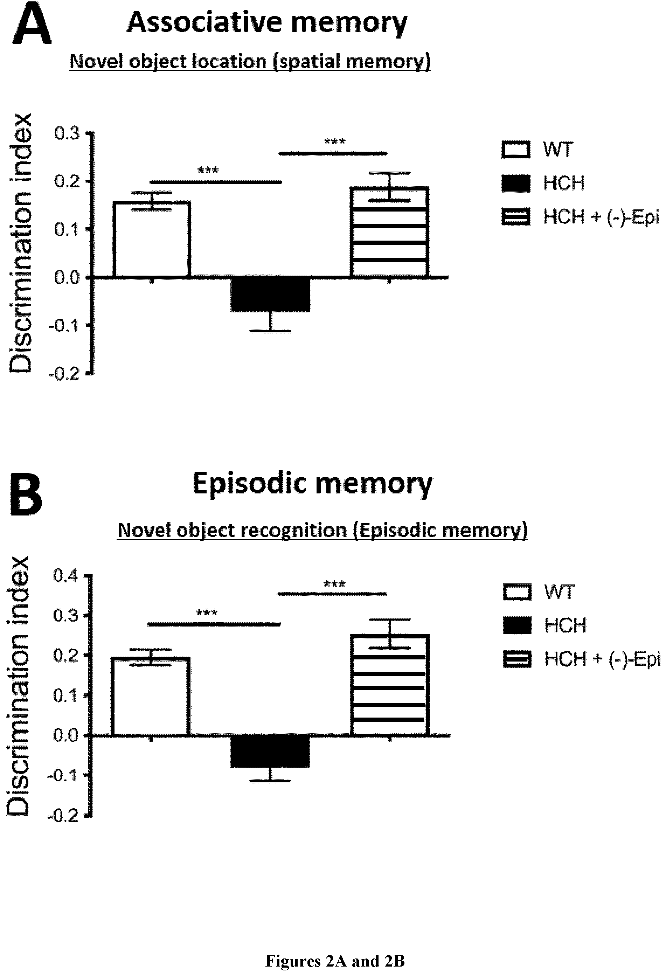

**Figure 2****: Learning impairment is reversed by subcutaneous injection of (-)-epicatechin daily in Hypochondroplasia (HCH) mouse model. A.** Novel object location (NOL) was performed in 4 months old male animals. Discrimination index was measured 24h after training phase to assess memory performance of Fgfr3 ^{N534K/+} and their control littermates. **B.** Novel object recognition (NOR) was performed in 4 months old male animals. Discrimination index was measured 24h after training phase to assess memory performance of *Fgfr3*^{*N534K*/}*⁺ +* (-)-Epi, *Fgfr3*^{*N534K*/*+*} and their control littermates. NOR and NOL were performed on *Fgfr3*^{*N534K*/*+*} (n=9) *Fgfr3*^{*N534K*/}*⁺ +* (-)-Epi (n=10) and their controls (n=15).

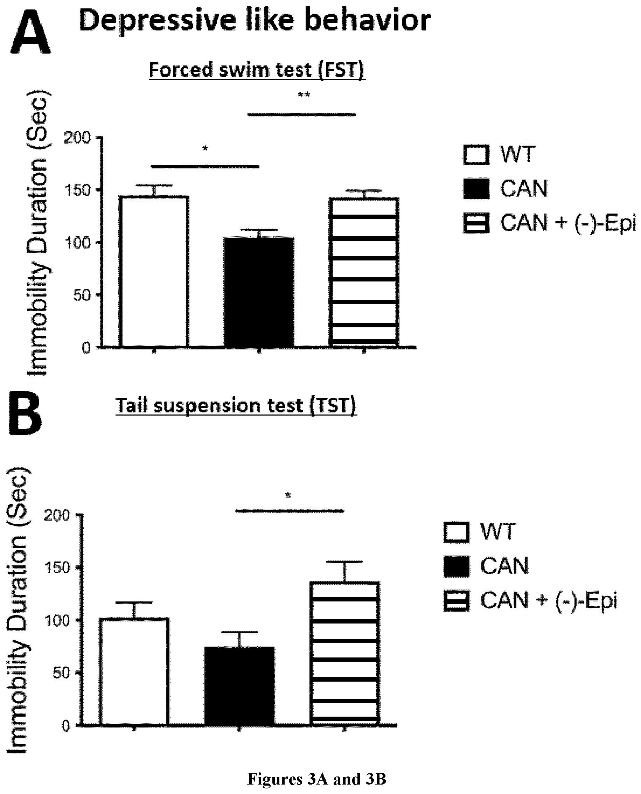

Figure 3: **Learning impairment and antidepressant-like behavior in Crouzon with acanthosis Nigricans (CAN) mouse model. A.** Forced swim test (FST) performed in 4 month-old male animals in two independent groups. FST was performed during two consecutive days. Immobility duration was assessed for both day 1 and day 2. (*Fgfr3*^{*+*/}*⁺,* n=9; *Fgfr3*^{*A385E*/*+,*} n=4, Fgfr3 ^{A385E/+} + (-)-Epi n=5). **B.** Tail suspension test (TST) performed in 4 month-old male animals during two consecutive days and immobility duration was assessed for both (*Fgfr3*^{*+*/}*⁺,* n=9; *Fgfr3*^{*A385E*/*+,*} n=4, *Fgfr3*^{*A385E*/*+*} + (-)-Epi n=5).

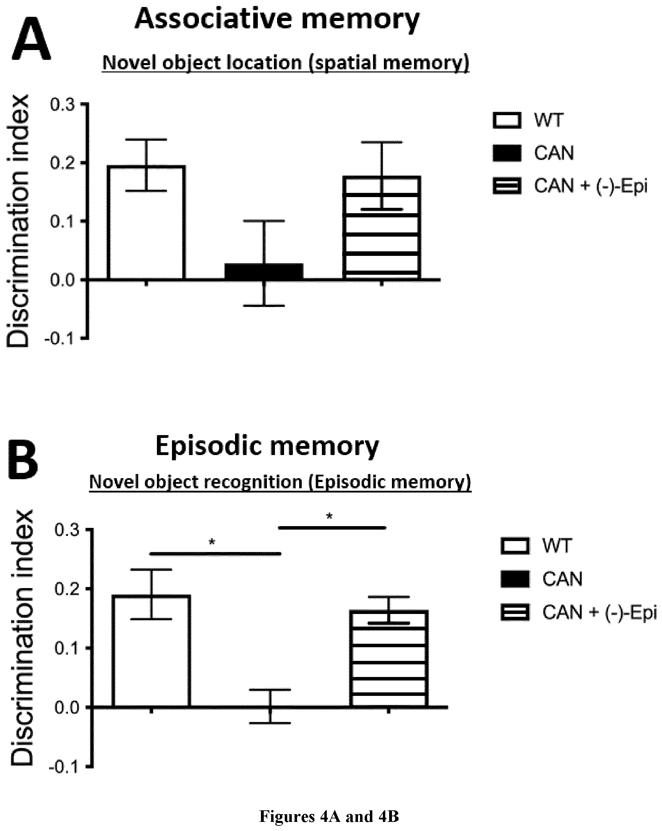

**Figure 4****: Learning impairment is reversed by subcutaneous injection of (-)-epicatechin daily in Crouzon with acanthosis Nigricans (CAN) mouse model. A.** Novel object location (NOL) was performed in 4 months old male animals. Discrimination index was measured 24h after training phase to assess memory performance of *Fgfr3*^{*A385E*/*+*} and their control littermates. **B.** Novel object recognition (NOR) was performed in 4 months old male animals. Discrimination index was measured 24h after training phase to assess memory performance of *Fgfr3*^{*A385E*/}*⁺ +* (-)-Epi, *Fgfr3*^{*A385E*/*+*} and their control littermates. NOR and NOL were performed *Fgfr3*^{*+*/}*⁺,* n=9; *Fgfr3*^{*A385E*/*+,*} n=4, *Fgfr3*^{*A385E*/*+*} + (-)-Epi n=5.

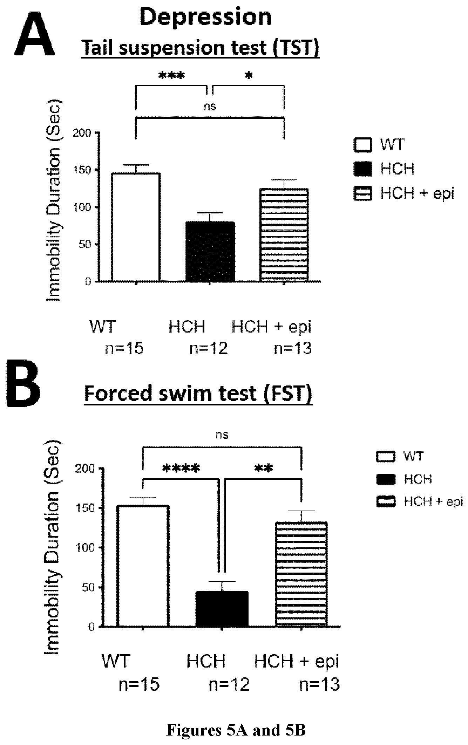

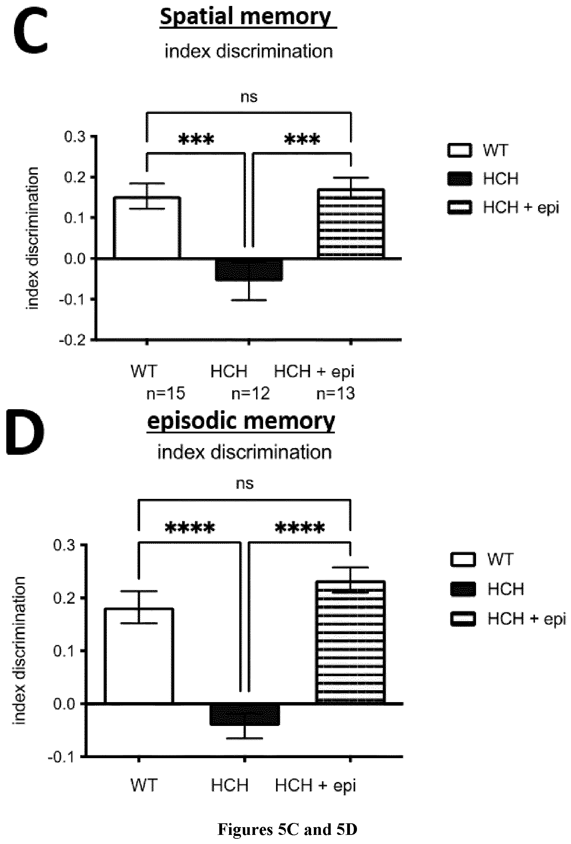

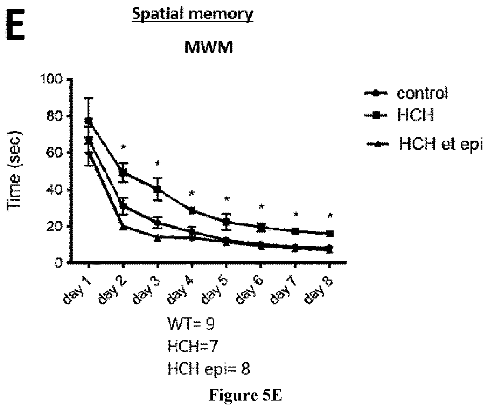

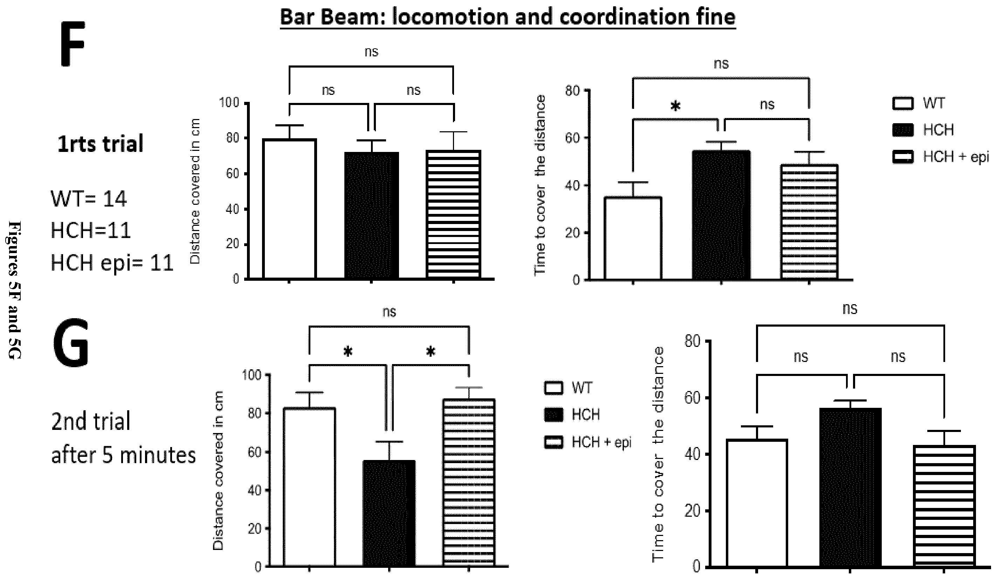

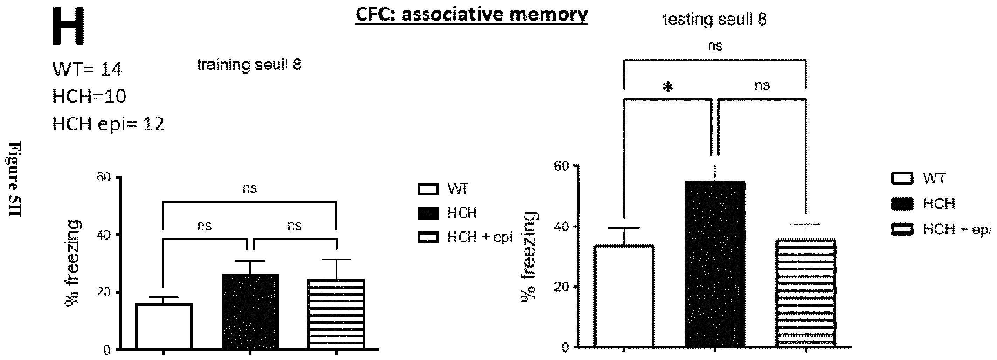

**Figure 5****. Learning impairment is reversed by bottle treatment of (-)-epicatechin daily in Hypochondroplasia (HCH) mouse model. A.** Tail suspension test (TST) was assessed for *Fgfr3*^{*+*/}*⁺,* n=15; *Fgfr3*^{*N534K*/*+,*} n=12, *Fgfr3*^{*N534K*/*+*} + (-)-Epi, n=13). **B.** Forced swim test (FST). was assessed for *Fgfr3*^{*+*/}*⁺,* n=15; *Fgfr3*^{*N534K*/*+,*} n=12, *Fgfr3*^{*N534K*/*+*} + (-)-Epi, n=13)**C**. Spatial memory (NOL) was assessed for *Fgfr3*^{*+*/}*⁺,* n=15; *Fgfr3*^{*N534K*/*+,*} n=12, *Fgfr3*^{*N534K*/*+*} + (-)-Epi, n=13). **D.** Episodic memory (NOR) was assessed for *Fgfr3*^{*+*/}*⁺,* n=15; *Fgfr3*^{*N534K*/*+,*} n=12, *Fgfr3*^{*N534K*/*+*} + (-)-Epi, n=13). **E.** Spatial memory (MWM) was assessed for *Fgfr3*^{*+*/}*⁺,* n=9; *Fgfr3*^{*N534K*/}*^{+,} n=7, Fgfr3*^{*N534K*/*+*} + (-)-Epi, n=8). **F and G.** Bar Beam: locomotion and coordination fine was assessed for *Fgfr3*^{*+*/}*⁺,* n=14; *Fgfr3*^{*N534K*/*+,*} n=11, *Fgfr3*^{*N534K*/*+*} + (-)-Epi, n=11). **H.** Associative memory (CFC) was assessed for *Fgfr3*^{*+*/}*⁺,* n=14; *Fgfr3*^{*N534K*/*+,*} n=10, *Fgfr3*^{*N534K*/*+*} + (-)-Epi, n=12).

### DEFINITION:

As used herein, the term **"FGFR3^{A385E/+}"** relates to a CAN mouse model in which a defective memory was observed.

As used herein, the term **"FGFR3^{N534K/+}"** relates to a HCH mouse model. The mutant mice display the clinical features of HCH with growth defects, growth plate anomalies, partial loss of synchondrosis and lordosis.

As used herein, the term **"FGFR3^{Y367C/+}"** relates to a mouse model that recapitulates the human ACH phenotype. The clinical hallmarks of ACH (e.g. dwarfism, associated with reduced size of the foramen magnum, hypoplasia of the mandibles, hearing loss, anomalies of the intervertebral discs and impairment of the ciliogenesis (Pannier et al. 2009, 2010, Mugniery et al 2012, Di Rocco et al 2014, Biosse Duplan et al 2016, Komla Ebri et al 2016, Martin et al 2018).

### EXAMPLE 1: METHODS

All procedures were approved by the French Animal Care and Use Committee. Genomic DNA was isolated from tail using NucleoSpin Tissue kit (Macherey-Nagel) according the manufacturer's instructions. The mice were genotyped using the following primers: 5'-GTGGGGGTTCTGCGGTTGG-3' (SEQ ID NO: 2) and 5'-TGACAGGCTTGGCAGTACGG-3' (SEQ ID NO: 3) for isolate WT and mutant mice. For all analyses, wild-type littermates were used as controls.

### Brain MRI

Mice brain MRI were acquired at the Small Animal Imaging Platform (Faculté de Médecine, Université Paris Descartes Sorbonne Paris Cité, Paris, France) using a small-animal 4.7-T MR imaging unit (Biospec 47/40; Bruker, Billerica, MA, USA) with a resolution of 100µm. *Fgfr3*^{*A385E*/*+*} mice and 5 *Fgfr3*^{*N534K*/*+*} male mice and 5 controls littermates of 4 months-of-age were anesthetized with isoflurane gas inhalation during acquisition. Three-dimensional reconstruction and measurement were performed using Imaris (Bitplane).

### Surgery and Drug treatment

4 months old *Fgfr3*^{*N534K*/*+*} mice and *Fgfr3*^{*A385E*/*+*} mice received daily sub-cutaneous administration of (-)-epicatechin (0. 15mg/kg) or vehicle (HcL 3,5mM, DMSO 2%) for at least seven days. For habituation and testing NOR and NOL, injection was performed 1 hour before testing phase. Timing of injection for the different tests : NOL at 7 days, NOR at 14 days, TST: at 22 days and FST at 24 days.

### Novel object recognition paradigm (NOR)

We used a modified version of the NOR task as described in Glatigny et al., 2019. Mice were transported a short distance from the holding mouse facility to the testing room in their home cages and left undisturbed for at least one hour before the beginning of the test. The testing room was lit with two 60-W light bulbs and behavior sessions were recorded with a camera above the testing arena (grey plastic box (60 × 40 × 32 cm)). Mice could not contact or see each other during the exposures. The light intensity was equal in all parts of the arena (approximately 20 lx). Two different objects were used, available in triplicate. The objects were a blue ceramic pot (diameter 6.5 cm, maximal height 7.5 cm) and a clear, plastic funnel (diameter 8.5 cm, maximal height 8.5 cm). The objects that serve as a novel object, as well as the left/right localization of the objects, were counterbalanced within each group. The objects elicited equal levels of exploration as determined in pilot experiments and training phase. Between exposures, mice were held individually in standard cages, the objects and arenas were cleaned with phagosphore, and the bedding replaced.

The NOR paradigm consists of three phases (over 3 days): a habituation phase, a training phase, and a testing phase. Mice were always placed in the center of the arena at the start of each exposure. On day 1: the habituation phase, mice were given 5 min to explore the arena, without any objects and were then taken back to their home cage. On day 2, the training phase, mice were allowed to explore, for 10 min, two identical objects arranged in a symmetric opposite position from the center of the arena and were then transported to their home cage. On day 3, the testing phase, mice were given 15 minutes to explore two objects: a familiar object and a novel one, in the same arena, keeping the same object localization.

The following behaviors were considered as exploration of the objects: sniffing, licking, or touching the object with the nose or with the front legs or directing the nose to the object at a distance ≤ 1 cm. Investigation was not scored if the mouse was on top of the object or completely immobile. The discrimination index was calculated as (time spent exploring the new object - time spent exploring the familiar object) / (total time spent exploring both objects). As a control, preference index for the (right/left) object location or for the object A versus B during the training phase of the novel object recognition (NOR) was measured in all groups of mice exposed to the test. We confirm here that no initial preference for any exposed object (A or B) or any orientation (right/left) was observed in any groups. The locomotion was assessed for each mouse. Behavior was scored on videos by two observers blind to treatment and the total exploration time of the objects was quantified in the testing phases.

### Novel object location (NOL)

For the novel object location task, all procedures were identical to the novel object recognition task except that during the testing phase, rather than presenting a novel object, mice encountered both familiar objects, with one object located in a different place in the arena. The time and frequency of exploration of the novel/relocated object is measured as an index of memory. Behavior was scored on videos by two observers blind to treatment and the total exploration time of the objects was quantified in the testing phases.

### Light-to-Dark Transition test (D/LT)

This test is based on the innate aversion of rodents to brightly illuminated areas and on their spontaneous exploratory behavior in response to the stressor that light represents. The test apparatus consists of a dark, safe compartment and an illuminated, aversive one. The lit compartment was brightly illuminated with an 8 W fluorescent tube (1000 lx). Naive mice were placed individually in the testing chamber in the middle of the dark area facing away from the doorway to the light compartment. Mice were tested for 10 min, and two parameters were recorded: time spent in the lit compartment and the number of transitions between compartments, indices of anxiety-related behavior and exploratory activity. Behavior was scored using an infrared light beam activity monitor using actiMot2 Software (PhenoMaster Software, TSE).

### Open Field Test (OFT)

This test takes advantage of the aversion of rodents to brightly lit areas. Each mouse is placed in the center of the OFT chamber (43 × 43 cm chamber) and allowed to explore for 30 min. Mice were monitored throughout each test session by infrared light beam activity monitor using actiMot2 Software (PhenoMaster Software, TSE). The overall motor activity was quantified as the total distance travelled (ambulation). Anxiety was quantified by measuring the time and distance spent in the center versus periphery of the open-field chamber.

### Tail suspension test (TST)

This test is based on the observation that rodents, after initial escape-oriented movements, develop an immobile posture when placed in an inescapable stressful situation. Each mouse is hung in an uncontrollable fashion by their tail at above 25 cm from the floor. Mice were tested for 5 min, and the time spent immobile was quantified.

### Forced swim test (FST)

This test is based on a similar observation than the TST. Each mouse is placed in a cylinder (height: 25 cm and diameter: 10 cm) filled with water (23-25°C). Mice were tested for 5 min, and the time spent immobile (behavioral despair) was quantified.

### Morrys Water Maze

The Morris water maze (MWM) is a test of spatial learning for mice that relies on distal cues to navigate from a start location to locate a submerged escape platform. Spatial learning is assessed across repeated trials during 8 days and reference memory is determined by location of the platform area.

### Bar beam

The bar beam is a test to measure fine motor coordination and balance in mice by assessing if the mice stay upright and walk across an elevated narrow beam to a safe platform. This test takes place over 2 consecutive days and we measured the time it takes for the mouse to traverse the beam and the maximum distance done

### EXAMPLE 2: HCH MODEL

### Clinical features of Hypochondroplasia

Existing literature indicates people with hypochondroplasia have an increased risk for learning disabilities and mental retardation. Learning disabilities may be present in an estimated 50 percent of people and may not be noticeable until the child is of school age. Cognitive disabilities are present in 10-12 percent of children. There are multiple areas of possible structural brain abnormalities, including enlarged temporal lobes, hippocampal dysplasia and temporal lobe dysgenesis (Bober MB et al., 2013., Linnankivi T et al., 2012; Philpott CM et al., 2013) HCH patients present lobe dysgenesis (Kannu et al. 2005) and abnormal hippocampus configuration (Linnankivi et al. 2012). Moreover, HCH patients present learning impairment, mild intellectual disability, global development delay and occasionally seizure and epilepsy (Linnankivi et al. 2012).

### Fgfr3^{N534K/}⁺ mice exhibit brain morphological abnormalities

We studied the *Hch* mouse model *Fgfr3*^{*Asn534Lys*/*+*} expressing the most common HCH mutation, p.Asn540Lys localized in FGFR3 tyrosine kinase I domain. In the central nervous system, FGFR3 is highly expressed in the hippocampus, a brain structure essential for memory integration mechanisms. We described that *Fgfr3*^{*N534K*/*+*} mice show skulls anomalies with a large skull and premature fusion of skull base synchondrosis. It was reported that brain anomalies and lobe dysgenesis are characteristics of Fgfr3 gain-of -function mutation mouse models. Therefore, we performed MRI and 3D reconstruction of *Fgfr3*^{*N534K*/*+*} mice and their control littermate brain. While we did not observe any significant abnormalities of hippocampal (p= 0,6905) and total brain volume (p= 0,3810) in HCH mice **(Data not shown),** MRI analyses showed modification of the brain shape when compared to controls **(Data not shown).**

### Learning and memory deficits in Fgfr^{N534K/}⁺ mice

Adult neurogenesis is known to play an important role in the maintenance of hippocampal memory capacity. We patiented 4 months-old male *Fgfr3*^{*Asn534Lys*/*+*} mice and their control littermate to series of behavioral tests measuring spatial (novel object location, NOL) and episodic (novel object recognition, NOR) learning and memory. In NOR, we found that Hch mutant mice explored significantly less (p< <0,0001) the novel object than control littermates during the testing phase **(Data not shown).** The same impairments were observed when spatial memory was analyzed through the NOL test. Indeed, we found that *Fgfr3*^{*N53K*/*+*} mice explored significantly less the relocated object than control littermates during the testing phase (p<0,0001) **(Data not shown).**

### Stress and memory behavior test

We performed on 4-month-old male mice a test related to the hippocampus associative memory (one-trial contextual fear conditioning, CFC). This test allows us to understand the ability of mice to remember an event associated with memory integration. During this behavorial test, we assess the freezing time of the mice, the mutant mice baseline was similar to control littermates during the training phase (p= 0,2303). However, during the testing day, *Fgfr3*^{*Asn534Lys*/*+*} mice show an increase context elicited freezing time compared to the control mice (p= 0,0466) indicating that contextual fear memory is exacerbated in the mutant mice **(Data not shown).**

### Anti-depressant effect of FGFR3

FGF pathway might be involved in depression disorders. Therefore, we performed Tail suspension test (TST) and force swim test (FST) in *Fgfr3*^{*N534K*/*+*} mice **(****Figures 1A and 1B****).** We treated *Fgfr3*^{*N534K*/*+*} mice for seven days at least with (-)-epicatechin. Animals patiented to the short-term and inescapable stress of being suspended by their tails or forced to swim will develop an immobile posture characteristic of depression-related behavior that will be scored. Performing these tests, we found that, either in TST or FST, *Fgfr3*^{*Asn534Lys*/*+*} mice present a reduction in immobile posture, suggesting that gain-of-function mutation in FGFR3 may have anti-depressant effects.

### The (-)-epicatechin treatment is sufficient to reverse cognitive impairments observed in Fgfr3^{Y367C/}⁺ mice

Subcutaneous injection of (-)-epicatechin is sufficient to abolish or eliminating the functional activation of the FGFR3 downstream signalling pathway and to restore the impairment of the ciliogenesis in vivo and/or in vitro in a Fgfr3 mouse model (*Fgfr3*^{*Y367C*/*+*} ) (De la Luz Cadiz-Gurrea M et al 2019, Martin L et al 2021). To confirm the impact of FGFR3 gain-of-function in learning impairment, we treated *Fgfr3*^{*N534K*/*+*} mice for seven days at least with (-)-epicatechin. As a result, we found that subcutaneous injections of (-)-epicatechin are sufficient to restore the spatial (NOL) **(****Figure 2A****)** and episodic memory deficits (NOR) **(****Figure 2B****)** observed in Fgfr ^{*N534K*/*+*} mice. Indeed, injected mutant mice were able to explore the relocated object (NOL) (p= 0,9538) and the novel object (NOR) (p= 0,8697) to the same levels than control littermates (p= 0,9538) **(****Figures 2A and 2B****).** Taken together these results confirm the importance of FGFR3 in the regulation of learning and memory. The rescue of these behavioral anomalies with (-)-epicatechin confirmed our hypotheses and supported the fact that FGFR3 over-activation was involved in the cognitive phenotype of patients with FGFR3-related chondrodysplasia.

### EXAMPLE 3: CAN MODEL

### Clinical features of Crouzon syndrome with acanthosis nigricans

CAN syndrome, associated to a FGFR3 mutation, exhibits a skeletal phenotype similar to Crouzon syndrome [MIM 123500] due to FGFR2 mutations (Coll et al., 2018, 2016; Di Rocco et al., 2011): oculo-orbital disproportion, prognathism, midfacial hypoplasia ***(Data not shown),*** and brachycephaly, secondary to a premature fusion of the coronal and sagittal sutures (at various degrees) ***(Data not shown).*** Skull vault anomalies exert mechanical pressure on the brain and increase the risk for elevated intracranial pressure (Al-Namnam et al., 2019) **(*Data not shown*)*.*** Additionally, brain MRI revealed mild temporal anomalies in three non-related CAN patients. Affected cases presented thickened parahippocampal groove, with modification of angle of the structure **(*Data not shown*)*.*** One case presenting cloverleaf skull could not be interpreted.

Skull base anomalies in Crouzon syndrome patients, both FGFR2- and FGFR3-related, contribute anteriorly to midface hypoplasia and posteriorly to cranio-vertebral junction anomalies. Premature fusion of sphenooccipital synchondrosis is associated to shortened skull base, while premature fusion of intraoccipital synchondroses is associated with a narrowed foramen magnum in CAN patients **(*Data not shown*)*.***

### Skeletal phenotype is mildly affected in CAN mouse model Fgfr3^{A385E/+}

To evaluate the effect of CAN mutation on the skeleton, we generated a mouse model expressing a ubiquitous p.Ala385Glu missense mutation corresponding to the p.Ala391Glu human mutation **(*Data not shown*)*.*** The FGFR3 p.Ala385Glu transcripts were detected in fibroblasts and calvarial osteoblasts **(*Data not shown*)*.*** Similarly, to the human disease, no obvious limb shortening phenotype was observed in *Fgfr3*^{*A385E*/*+*} mice from antenatal and newborn stages (data not shown) to adult stage **(*Data not shown*)*.*** Body weight, nasal-anal and femurs and tibias lengths were similar in *Fgfr3*^{*A385E*/}*⁺and Fgfr3*^{+/+} mice **(*Data not shown*)*.*** Normal femur growth was confirmed by cartilage assessment in *Fgfr3*^{*A385E*/*+*} showing well organized growth plate without anomaly of the hypertrophic zone revealed with collagen type X staining **(*Data not shown*).** To confirm the absence of abnormal phenotype, bone structure parameters were assessed. Micro CT images of 3 months old femurs revealed a normal structure of the trabecular and cortical bone in *Fgfr3*^{*A385E*/*+*} mice **(*Data not shown*)*.***

The craniofacial phenotype was assessed using micro CT skull acquisition. The *Fgfr3*^{*A385E*/*+*} mice showed normal craniofacial features **(*Data not shown*);** the coronal sutures and skull base synchondroses were patent at post-natal day 21 similarly to control mice **(*Data not shown*)*.*** Landmark-based geometric morphometrics (Heuzé et al., 2010) of *Fgfr3*^{*A385E*/*+*} mice and *Fgfr3*^{*+*/*+*} mice did not show any difference in skull shape (d = 0.0148; p = 0.0940). However, the shape of the mandible was significantly different between the two groups of mice (d=0.0159; P<0.01) **(*Data not shown*)*.*** In order to confirm the absence of premature closure of the fronto-parietal suture, we assessed in vitro function of calvarial *Fgfr3*^{*A385E*/*+*} osteoblasts. No significant differences in mineralization capacity, proliferation and Mitogen-activated protein kinases (MAPK) activation were observed in the osteoblasts of *Fgfr3*^{*A385E*/*+*} mice compared to controls **(*Data not shown*)*.*** All these data allowed us to conclude that p.Ala385Glu mutation expressed in osteoblasts was not active and consequently did not affect craniofacial development **(*Data not shown*)*.*** These data explain the absence of craniosynostosis phenotype in *Fgfr3*^{*A385E*/*+*} mice. Regarding the skin, no signs of hyperkeratosis nor modification in thickness and pigmentation of the epidermis was detected in *Fgfr3*^{*A385E*/*+*} mice **(*Data not shown*)*.***

### Fgfr3^{A385E/}⁺ mouse model show dentate gyrus decreased neurogenesis

Structural brain anomalies have been described in CAN (Gürbüz et al., 2016) **(*Data not shown*)** and Muenke patients (Abdel-Salam et al., 2011; Grosso et al., 2003; Okubo et al., 2017) . These anomalies include abnormal morphology of hippocampus and temporal lobes. It is well known that the premature fusion of cranial sutures modifies the shape of the skull and impair the normal brain growth, causing functional issues such as increased intracranial pressure, visual impairment, deafness and cognitive impairment (Di Rocco et al., 2011). Previous studies showed that patients with MS presented deficits in adaptive and executive functions. This behavior phenotype included working memory deficits, attention-deficit hyperactivity disorder, emotional control and anxiety (Yarnell et al., 2015). These neurological impairments suggest an impact of FGFR3 in the brain for the control of cognitive functions. Hence, we performed magnetic resonance imaging (MRI) in *Fgfr3*^{*A385E*/*+*} mice at four months of age and measured the volumes of various brain regions.

No modifications of the volume and any compression were observed in the various brain regions of *Fgfr3*^{*A385E*/*+*} mice suggesting normal embryonic brain development **(*Data not shown*)*.*** However, FGF and FGFR are known to be involved in proliferation and differentiation of neural stem cells and neural progenitors in the central nervous system (Huang et al., 2017; Kang and Hébert, 2015, Moldrich et al., 2011; Ohkubo et al., 2004; Stevens et al., 2012). Therefore, we hypothesized that the *Fgfr3^{A385E}* mutation expressed in the brain of *Fgfr3*^{*A385E*/*+*} mice could impact adult neurogenesis. The absence of craniofacial anomalies is a good opportunity to assess neurogenesis specifically during adult period. Exploring the role of *Fgfr3* in adult neurogenesis, we observed a similar expression of FGFR3 in *Fgfr3*^{*A385E*/*+*} and *Fgfr3*^{*+*/*+*} mouse hippocampi by immunofluorescence **(*Data not shown*)** and western blotting **(*Data not shown*).** However, the canonical MAPK pathway activated by FGFR3 is found dysregulated in both Fgfr3 knock in mouse model (Komla-Ebri et al., 2016) and FGFR3 knock out mouse model (Zhou et al., 2015). Indeed, we observed a significant increased expression of phosphorylated Erk1/2 in adult hippocampi lysates **(*Data not shown*)** thus confirming that the *Fgfr3^{A385E}* mutation in the brain activated the MAPK pathway. FGFR3 play a key role in progenitor cell proliferation and neuronal differentiation in dentate gyrus (Inglis-Broadgate et al., 2005; Kang and Hébert, 2015; Moldrich et al., 2011; Thomson et al., 2009). In 4 months old *Fgfr3^{A385E}* hippocampi, using NeuN marker, we showed that mature neuron area of dentate gyrus granular layer is significantly decreased compared to control **(*Data not shown*)*.*** We assessed whether this decreased neuronal population was caused by decreased progenitor proliferation. The positive cell number for the cell cycle KI67 marker was significantly reduced in the dentate gyrus subgranular zone in *Fgfr3*^{*A385E*/*+*} mice **(*Data not shown*)*.*** In the granular zone, the neuronal differentiation rate revealed by doublecortin (DCX) immunolabelling is slightly decreased **(*Data not shown*)*.*** Altogether these data strongly suggested that the Fgfr3 gain-of-function mutation affects predominantly proliferation thus impacting neuronal mature differentiation in dentate gyrus.

### Fgfr3^{A385E/}⁺ mouse model show decreased learning capacity and antidepressant effect

Reduced size of hippocampal structures and reduced proliferation were showed to be associated with memory and cognitive alterations in human and mouse (Kitamura and Inokuchi, 2014). Therefore, we patiented 4-month-old *Fgfr3*^{*A385E*/*+*} mice and their control littermates to a series of behavioral tests, that is thought to reflect behavioral functions related to hippocampus, measuring associative (one-trial contextual fear conditioning, CFC) and episodic (novel object recognition test, NOR), and spatial (Morris Water Maze, MWM) learning and memory **(****Figures 4A and 4B****).** In CFC, mutant mice exhibited no differences in baseline freezing time. However, Fgfr3 gain-of-function mutation resulted in decreased context-elicited freezing time during the testing phase compared to their control littermates, indicating that contextual fear memory is impaired in the mutant mice **(*Data not shown*)*.***

Next, we used a modified version of the NOR paradigm (Denny et al., 2012; Ennaceur and Delacour, 1988) that measures the rodent's ability to recognize a novel object in the environment. Wild type mice are capable of differentiating novel objects from familiar ones and tend to explore novel ones for longer time. As shown in **(****Figures 4A** **and AB),** 4-month-old *Fgfr3*^{*A385E*/*+*} mice explored significantly less the novel object than controls. However, no impairments were observed when memory was analyzed through the MWM task (assessing spatial learning and memory in rodents). Of note, *Fgfr3*^{*A385E*/*+*} mice and control had comparable performance in the open field test (OFT) and Light/Dark paradigm (L/DT) ***(Data not shown*)*,*** indicating that their locomotion and anxious state were intact.

Next, we evaluated the coping strategy to an inescapable stress using the Forced Swim Test (FST) and the Tail suspension test (TST). As shown in **(****Figure 3A****),** 4-month-old *Fgfr3*^{*A385E*/*+*} mice spend significantly less time immobile than control mice during the FST. The same impairments were observed during the TST. Indeed, mutant mice spend significantly less time immobile than control littermates **(****Figure 3B****).**

Taken together, these data demonstrate that Fgfr3 gain-of-function mutation significantly influences hippocampal-dependent episodic memory and associative fear memory acquisition and affects the coping strategy to an inescapable stress.

Importantly, these data indicate that *Fgfr3^{A385E}* mutation in mice reproduced the behavioral deficits previously described in human patient, including working memory deficits, and emotional control and attention-deficit hyperactivity disorder (Yarnell et al., 2015).

### (-)-epicatechin subcutaneous injections rescue the cognitive impairments of Fgfr3^{A385E/}⁺ mice

To confirm that cognitive impairments in *Fgfr3*^{*A385E*/*+*} mice were due to increased phosphorylation of the receptor and activation of several downstream signaling pathways, we decided to treat the mice with (-)-epicatechin (De la Luz Cadiz-Gurrea M et al 2019, Martin L et al 2021). 4-month-old *Fgfr3*^{*A385E*/*+*} mice and their control littermates received subcutaneous injections for 7 days at least with (-)-epicatechin or vehicle solution and were submitted to two behavioral tests (NOR and FST; **Figures 4B** **and** **3A****).** Injections of (-)-epicatechin reversed the memory deficits observed in the NOR paradigm for the *Fgfr3*^{*A385E*/*+*} mice **(****Figure 4B****)** and reestablished the coping strategy to an inescapable stress observed in the FST **(****Figure 3A****)** compared to the control littermates. The absence of craniocerebral disproportion, and thus the lack of potentially increased intracranial hypertension in *Fgfr3*^{*A385E*/*+*} mice allow us to conclude that the reported behavioral anomalies observed were due to the direct impact of *Fgfr3^{A385E}* mutation on the brain. The rescue of these behavioral anomalies with (-)-epicatechin confirmed our hypotheses and supported the fact that FGFR3 over-activation was involved in the cognitive phenotype of patients with FGFR3-related craniosynostoses.

### EXAMPLE 4: BOTTLE TREATMENT

### (-)-epicatechin oral administration rescue the cognitive impairments of Fgfr3^{N534K} mice

To confirm the direct implication of brain FGFR3 in cognitive disorders observed in *Fgfr3*^{*N534K*/}*⁺ mice,* we decided to treat mice with (-)-epicatechin bottle treatment. The animals were treated with bottle (-)-epicatechin (3mg/L) during 4 weeks before behavioural tests. (-)-epicatechin was selected for its highest binding specificity for FGFR3.

Here, bottle (-)-epicatechin treatment in adult *Fgfr3*^{*N534K*/}*⁺ mice* (hypochondroplasia mouse model) showed rescue of the spatial (NOL; Morrys Water Maze, **Figures 5C-5E****),** episodic and associative (CFC, **Figure 5H****)** memory deficits, antidepressant (TST, FST, **Figures 5A and 5B****)** effect and locomotion and coordination fine (Bar beam, **Figures 5F and 5G****).** These data demonstrated the direct impact of the Fgfr3 gain-of-function mutation in the brain on cognitive performances. We also demonstrated that FGFR3 played a major role in hippocampal adult neurogenesis and we established a direct link between hippocampal anomalies and learning and stress response.

These results highlighted the interests of catechin for the treatment of the cognitive impairments in subjects harboring a FGFR3 gain-of-function mutation (FGFR3-related disorders = achondroplasia, hypochondroplasia, Crouzon syndrome with acanthosis nigricans and Muenke syndromes) and disorders with defective memories.

### Discussion

CAN syndrome is a very rare syndromic craniosynostosis associated to the specific p.Ala391Glu gain-of-function mutation in FGFR3 (Meyers et al., 1995). The effect of the p.Ala391Glu mutation was previously described as causing overactivation of FGFR3 (Chen et al., 2013, 2011; Li et al., 2006).

Mouse *Fgfr3*^{*A385E*/*+*} CAN model showed an absence of major craniofacial skeletal phenotype. Interestingly, the phenotype of *Fgfr3*^{*A385E*/*+*} was comparable to the one observed in *Fgfr3*^{*P244R*/*P244R*} mice, a mouse model for Muenke syndrome. Suture and synchondroses were found to be mildly affected in Muenke *Fgfr3*^{*P244R*/*P244R*} mutants, with coronal suture fused in a minority of individuals (Laurita et al., 2011; Twigg et al., 2008).

In CAN and Muenke, premature fusion of skull vault sutures combined with premature fusion of skull base synchondroses associated to narrowing of foramen magnum lead to increased intracranial pressure in patients (Di Rocco et al., 2011). Premature fusion of the skull vault is also associated with brain structures anomalies, including abnormal hippocampus development (Grosso et al., 2003; Giirbiiz et al., 2016; Okubo et al., 2017).

HCH patients (14600 MIM) are characterized by rhizomelic dwarfism, mild macrocephaly, hypoplasia of the midface, short square ilia and in some case acanthosis nigricans (Blomberg et al. 2010). The most common HCH mutation (p.Asn540Lys) is localized in tyrosine kinase 1 domain of FGFR3 (Bonaventure et al. 1996; Rousseau et al. 1994).

HCH patients present lobe dysgenesis (Kannu et al. 2005) and abnormal hippocampus configuration (Linnankivi et al. 2012). Moreover, HCH patients present learning impairment, mild intellectual disability, global development delay and occasionally seizure and epilepsy (Linnankivi et al. 2012).

In Fgfr3 mouse models, previous studies on mouse model *Fgfr3*^{*+*/*K644E*} mutation associated to thanatophoric dysplasia, both ubiquitously and under Nestin promoter, presented severe overgrowth of cerebrum and cortex, while *Fgfr3*^{*-*/*-*} mouse presented an underdeveloped neocortex (Inglis-Broadgate et al., 2005; Moldrich et al., 2011; Thomson et al., 2009, 2007). It is commonly accepted that the premature fusion of cranial sutures observed in craniosynostoses modifies brain morphology and is associated with cognitive impairment via a chronic increase in intracranial pressure (Aldridge et al., 2010; Arnaud-López et al., 2007; Gürbüz et al., 2016; Martínez-Abadías et al., 2011). Here, taking advantage of the absence of abnormal skull phenotype in the *Fgfr3*^{*A385E*/*+*} mice, we analyzed the role of activating Fgfr3 gain-of-function mutation in the brain. The brain of *Fgfr3*^{*A385E*/*+*} mice did not present severe morphological modifications, thus indicating that *Fgfr3^{A385E}* mutation had a moderate impact on brain embryonic neurogenesis. By contrast, the analysis of adult hippocampal neurogenesis showed decreased progenitor proliferation in the dentate gyrus, supported by decreased granular zone of the dentate gyrus.

Interestingly, previous studies reported decreased progenitor proliferation in FGFR1,2,3 loss-of-function mutations, while over-activation of FGFR3 (*Fgfr3^{TDIIK650E}*) promote increased progenitor differentiation in the dentate gyrus (Kang and Hébert, 2015). Our results contrast with these observations. We observed that an over-activation of FGFR3 led to decreased cell proliferation in *Fgfr3*^{*A385E*/*+*} mice. It seemed that the level of phosphorylation of the receptor interfered with neurogenesis: *Fgfr3^{TDIIK650E}* mutation led to the excessive receptor activation levels whereas *Fgfr3^{A385E}* mutation led to a more moderate over-activation. These data suggested that FGFR regulation of hippocampal neurogenesis is linked to the level of FGFR3 activation.

We next analyzed the impact of *Fgfr3^{A385E}* and of *Fgfr3^{N534K}* mutation on brain cognitive functions. We observed that *Fgfr3*^{*A385E*/*+*} mice *Fgfr3*^{*N534K*/}*⁺ mice* exhibited severe impairments working and episodic memory functions without any locomotion, anxiety-related behavioral or spatial memory phenotype. While, the effect of FGFR signals on memory is unclear, to date, our study is the first to associate Fgfr3 mutation to cognitive abnormalities in mice. Mechanistically, the deficit in learning and memory performances could be linked, at least in part, to the decreased hippocampal neurogenesis observed in our mutant mice. Interestingly, the deletion of Fgfr2 in embryo and adult mice showed decreased progenitor proliferation and differentiation in the dentate gyrus with specific negative effect on associative and spatial memory capacity (Stevens et al., 2012). Collectively, our data demonstrate that Fgfr3 gain-of-function mutation lead to severe learning and memory deficits and lower adult neurogenesis in the hippocampus.

In addition, a decreased coping strategy to an inescapable stress (previously name "depression like behavior") was observed in *Fgfr3*^{*A385E*/*+*} mice and *Fgfr3*^{*N534K*/}*⁺ mice.* This role of FGFR3 was previously reported in major depressive disorders in human associated with downregulation of the receptor (Evans et al., 2004). Furthermore, patients with FGFR3 related craniosynostosis syndromes, showed an impairment of emotional control and anxiety behavior (de Jong et al., 2010; Maliepaard et al., 2014; Yarnell et al., 2015). Today, no study reported depressive or mood disorders in craniosynostoses cases. Animal models studies also demonstrated the antidepressant and anxiolytic effect of FGF2 in FGF2 knockout or exogenous FGF2 injections (Elsayed et al., 2012; Salmaso et al., 2016). In contrast to FGF2, FGF9 plays a depressive effect in mouse (Aurbach et al., 2015). Both FGF2 and FGF9 are among the major FGFR3 ligands and can also bind the other FGFRs. These observations are in agreement with our data and could involve a complex combination of different FGFs and FGFR1, 2, 3 bindings.

To confirm the direct implication of brain FGFR3 in cognitive disorders observed in *Fgfr3*^{*A385E*/*+*} mice and *Fgfr3*^{*N534K*/}*⁺ mice,* we decided to treat mice with (-)-epicatechin, following selective brain injections. (-)-epicatechin was selected for its highest binding specificity for FGFR3 and a previous work showed the efficacy of (-)-epicatechin in skeletal anomalies in an FGFR3 related Achondroplasia mouse model (Martin et al 2021).

Here, subcutaneous injection of (-)-epicatechin in adult *Fgfr3*^{*A385E*/*+*} mice and *Fgfr3*^{*N534K*/}*⁺ mice* showed rescue of the working and episodic memory deficits and antidepressant effect. These data demonstrated the direct impact of the Fgfr3 gain-of-function mutation in the brain on cognitive performances. We also demonstrated that FGFR3 played a major role in hippocampal adult neurogenesis and we established a direct link between hippocampal anomalies and learning and stress response.

These results highlighted the interests of catechin for the treatment of the cognitive impairments in subjects harboring acFGFR3 gain-of-function mutation.

### REFERENCES:

Throughout this application, various references describe the state of the art to which this invention pertains.
Abdel-Salam, G.M.H., Flores-Sarnat, L., El-Ruby, M.O., Parboosingh, J., Bridge, P., Eid, M.M., El-Badry, T.H., Effat, L., Curatolo, P., Temtamy, S.A., 2011. Muenke syndrome with pigmentary disorder and probable hemimegalencephaly: An expansion of the phenotype. Am. J. Med. Genet. 155, 207-214. https://doi.org/10.1002/ajmg.a.33777
Aldridge, K., Hill, C.A., Austin, J.R., Percival, C., Martinez-Abadias, N., Neuberger, T., Wang, Y., Jabs, E.W., Richtsmeier, J.T., 2010. Brain phenotypes in two FGFR2 mouse models for Apert syndrome. Dev. Dyn. 239, 987-997. https://doi.org/10.1002/dvdy.22218
Al-Namnam, N.M., Hariri, F., Thong, M.K., Rahman, Z.A., 2019. Crouzon syndrome: Genetic and intervention review. J Oral Biol Craniofac Res 9, 37-39. https://doi.org/10.1016/;jobcr.2018.08.007
Arnaud-López, L., Fragoso, R., Mantilla-Capacho, J., Barros-Núñez, P., 2007. Crouzon with acanthosis nigricans. Further delineation of the syndrome. Clin. Genet. 72, 405-410. https://doi.org/10.1111/j.1399-0004.2007.00884.x
Aurbach, E.L., Inui, E.G., Turner, C.A., Hagenauer, M.H., Prater, K.E., Li, J.Z., Absher, D., Shah, N., Blandino, P., Bunney, W.E., Myers, R.M., Barchas, J.D., Schatzberg, A.F., Watson, S.J., Akil, H., 2015. Fibroblast growth factor 9 is a novel modulator of negative affect. Proc Natl Acad Sci U S A 112, 11953-11958. https://doi.org/10.1073/pnas.1510456112
Berton, Olivier, Colleen A. McClung, Ralph J. DiLeone, Vaishnav Krishnan, William Renthal, Scott J. Russo, Danielle Graham, et al. 2006. « Essential Role of BDNF in the Mesolimbic Dopamine Pathway in Social Defeat Stress ». Science 311 (5762): 864-68. https://doi.org/10.1126/science.1120972.
Blomberg, M., E. M. Jeppesen, F. Skovby, et E. Benfeldt. 2010. « FGFR3 Mutations and the Skin: Report of a Patient with a FGFR3 Gene Mutation, Acanthosis Nigricans, Hypochondroplasia and Hyperinsulinemia and Review of the Literature ». Dermatology 220 (4): 297-305. https://doi.org/10.1159/000297575.
Bonaventure, J., F. Rousseau, L. Legeai-Mallet, M. Le Merrer, A. Munnich, et P. Maroteaux. 1996. « Common Mutations in the Fibroblast Growth Factor Receptor 3 (FGFR3) Gene Account for Achondroplasia, Hypochondroplasia, and Thanatophoric Dwarfism ». American Journal of Medical Genetics 63 (1): 148-54. https://doi.org/10.1002/(SICI)1096-8628(19960503)63:1<148::AID-AIMG26>3.0.CO;2-N.
Chen, F., Degnin, C., Laederich, M., Horton, W., Hristova, K., 2011. The A391E mutation enhances FGFR3 activation in the absence of ligand. Biochim Biophys Acta 1808, 2045-2050. https://doi.org/10.1016/j.bbamem.2011.04.007
Chen, F., Sarabipour, S., Hristova, K., 2013. Multiple Consequences of a Single Amino Acid Pathogenic RTK Mutation: The A391E Mutation in FGFR3. PLoS One 8. https://doi.org/10.1371/journal.pone.0056521
Coll, G., Lemaire, J.-J., Di Rocco, F., Barthélémy, I., Garcier, J.-M., De Schlichting, E., Sakka, L., 2016. Human Foramen Magnum Area and Posterior Cranial Fossa Volume Growth in Relation to Cranial Base Synchondrosis Closure in the Course of Child Development: Neurosurgery 79, 722-735. https://doi.org/10.1227/NEU.0000000000001309
Coll, G., Sakka, L., Botella, C., Pham-Dang, N., Collet, C., Zerah, M., Arnaud, E., Di Rocco, F., 2018. Pattern of Closure of Skull Base Synchondroses in Crouzon Syndrome. World Neurosurgery 109, e460-e467. https://doi.org/10.1016/j.wneu.2017.09.208
Colvin, J.S., Bohne, B.A., Harding, G.W., McEwen, D.G., Ornitz, D.M., 1996. Skeletal overgrowth and deafness in mice lacking fibroblast growth factor receptor 3. Nat. Genet. 12, 390-397. https://doi.org/10.1038/ng0496-390
Commons, K.G., Cholanians, A.B., Babb, J.A., Ehlinger, D.G., 2017. The Rodent Forced Swim Test Measures Stress-Coping Strategy, Not Depression-like Behavior. ACS Chem Neurosci 8, 955-960. https://doi.org/10.1021/acschemneuro.7b00042
Cornille, Maxence, Stéphanie Moriceau, Roman Hossein Khonsari, Yann Heuzé, Anne Morice, Eric Arnaud, Corinne Collet, et al. 2020. « FGFR3 gain-of-function is associated to memory and learning deficit in Crouzon syndrome mouse model », under submission 2020.
de Jong, T., Bannink, N., Bredero-Boelhouwer, H.H., van Veelen, M.L.C., Bartels, M.C., Hoeve, L.J., Hoogeboom, A.J.M., Wolvius, E.B., Lequin, M.H., van der Meulen, J.J.N.M., van Adrichem, L.N.A., Vaandrager, J.M., Ongkosuwito, E.M., Joosten, K.F.M., Mathijssen, I.M.J., 2010. Long-term functional outcome in 167 patients with syndromic craniosynostosis; defining a syndrome-specific risk profile. J Plast Reconstr Aesthet Surg 63, 1635-1641. https://doi.org/10.1016/j.bjps.2009.10.029
Deng, C., Wynshaw-Boris, A., Zhou, F., Kuo, A., Leder, P., 1996. Fibroblast growth factor receptor 3 is a negative regulator of bone growth. Cell 84, 911-921.
Denny, C.A., Burghardt, N.S., Schachter, D.M., Hen, R., Drew, M.R., 2012. 4- to 6-week-old adult-born hippocampal neurons influence novelty-evoked exploration and contextual fear conditioning. Hippocampus 22, 1188-1201. https://doi.org/10.1002/hipo.20964
Di Rocco, F., Biosse Duplan, M., Heuzé, Y., Kaci, N., Komla-Ebri, D., Munnich, A., Mugniery, E., Benoist-Lasselin, C., Legeai-Mallet, L., 2014. FGFR3 mutation causes abnormal membranous ossification in achondroplasia. Hum. Mol. Genet. 23, 2914-2925. https://doi.org/10.1093/hmg/ddu004
Di Rocco, F., Collet, C., Legeai-Mallet, L., Arnaud, E., Le Merrer, M., Hadj-Rabia, S., Renier, D., 2011. Crouzon syndrome with acanthosis nigricans: a case-based update. Childs Nerv Syst 27, 349-354. https://doi.org/10.1007/s00381-010-1347-z
Elsayed, M., Banasr, M., Duric, V., Fournier, N.M., Licznerski, P., Duman, R.S., 2012. Antidepressant effects of Fibroblast Growth Factor-2 in behavioral and cellular models of depression. Biol Psychiatry 72, 258-265. https://doi.org/10.1016/j.biopsych.2012.03.003
Ennaceur, A., Delacour, J., 1988. A new one-trial test for neurobiological studies of memory in rats. 1: Behavioral data. Behav. Brain Res. 31, 47-59.
Evans, S.J., Choudary, P.V., Neal, C.R., Li, J.Z., Vawter, M.P., Tomita, H., Lopez, J.F., Thompson, R.C., Meng, F., Stead, J.D., Walsh, D.M., Myers, R.M., Bunney, W.E., Watson, S.J., Jones, E.G., Akil, H., 2004. Dysregulation of the fibroblast growth factor system in major depression. Proc Natl Acad Sci U S A 101, 15506-15511. https://doi.org/10.1073/pnas.0406788101
Gibbs, L., Legeai-Mallet, L., 2007. FGFR3 intracellular mutations induce tyrosine phosphorylation in the Golgi and defective glycosylation. Biochimica et Biophysica Acta (BBA) - Molecular Cell Research 1773, 502-512. https://doi.org/10.1016/j.bbamcr.2006.12.010
Glatigny, M., Moriceau, S., Rivagorda, M., Ramos-Brossier, M., Nascimbeni, A.C., Lante, F., Shanley, M.R., Boudarene, N., Rousseaud, A., Friedman, A.K., Settembre, C., Kuperwasser, N., Friedlander, G., Buisson, A., Morel, E., Codogno, P., Oury, F., 2019. Autophagy Is Required for Memory Formation and Reverses Age-Related Memory Decline. Curr. Biol. https://doi.org/10.1016/j.cub.2018.12.021
Grosso, S., Farnetani, M.A., Berardi, R., Bartalini, G., Carpentieri, M., Galluzzi, P., Mostardini, R., Morgese, G., Balestri, P., 2003. Medial temporal lobe dysgenesis in Muenke syndrome and hypochondroplasia. American Journal of Medical Genetics Part A 120A, 88-91. https://doi.org/10.1002/ajmg.a.10171
Gudernova, I., Vesela, I., Balek, L., Buchtova, M., Dosedelova, H., Kunova, M., Pivnicka, J., Jelinkova, I., Roubalova, L., Kozubik, A., Krejci, P., 2015. Multikinase activity of fibroblast growth factor receptor (FGFR) inhibitors SU5402, PD173074, AZD1480, AZD4547 and BGJ398 compromises the use of small chemicals targeting FGFR catalytic activity for therapy of short stature syndromes. Hum. Mol. Genet. https://doi.org/10.1093/hmg/ddv441
Gürbüz, F., Ceylaner, S., Topaloǧlu, A.K., Yiiksel, B., 2016. Crouzonodermoskeletal Syndrome with Hypoplasia of Corpus Callosum and Inferior Vermis. J Clin Res Pediatr Endocrinol 8, 373-374. https://doi.org/10.4274/jcrpe.3343
Heuzé, Y., Boyadjiev, S.A., Marsh, J.L., Kane, A.A., Cherkez, E., Boggan, J.E., Richtsmeier, J.T., 2010. New insights into the relationship between suture closure and craniofacial dysmorphology in sagittal nonsyndromic craniosynostosis. J Anat 217, 85-96. https://doi.org/10.1111/j.1469-7580.2010.01258.x
Huang, J.-Y., Lynn Miskus, M., Lu, H.-C., 2017. FGF-FGFR Mediates the Activity-Dependent Dendritogenesis of Layer IV Neurons during Barrel Formation. J. Neurosci. 37, 12094-12105. https://doi.org/10.1523/JNEUROSCI.1174-17.2017
Imai, Yoshinori, et Shinichi Kohsaka. 2002. « Intracellular Signaling in M-CSF-Induced Microglia Activation: Role of Iba1 ». Glia 40 (2): 164-74. https://doi.org/10.1002/glia.10149. Inglis-Broadgate, S.L., Thomson, R.E., Pellicano, F., Tartaglia, M.A., Pontikis, C.C., Cooper, J.D., Iwata, T., 2005. FGFR3 regulates brain size by controlling progenitor cell proliferation and apoptosis during embryonic development. Developmental Biology 279, 73-85. https://doi.org/10.1016/j.ydbio.2004.11.035
Itoh, Kyoko, Ritsuko Pooh, Yonehiro Kanemura, Mami Yamasaki, et Shinji Fushiki. 2013. « Brain Malformation with Loss of Normal FGFR3 Expression in Thanatophoric Dysplasia Type I ». Neuropathology 33 (6): 663-66. https://doi.org/10.1111/neup.12036.
Kang, W., Hébert, J.M., 2015. FGF Signaling Is Necessary for Neurogenesis in Young Mice and Sufficient to Reverse Its Decline in Old Mice. J Neurosci 35, 10217-10223. https://doi.org/10.1523/JNEUROSCI.1469-15.2015
Kannu, Peter, Ian M. Hayes, Simone Mandelstam, Leo Donnan, et Ravi Savarirayan. 2005. « Medial Temporal Lobe Dysgenesis in Hypochondroplasia ». American Journal of Medical Genetics. Part A 138 (4): 389-91. https://doi.org/10.1002/ajmg.a.30974.
Kitamura, T., Inokuchi, K., 2014. Role of adult neurogenesis in hippocampal-cortical memory consolidation. Mol Brain 7, 13. https://doi.org/10.1186/1756-6606-7-13
Klingenberg, C.P., 2011. MorphoJ: an integrated software package for geometric morphometrics. Mol Ecol Resour 11, 353-357. https://doi.org/10.1111/j.1755-0998.2010.02924.x
Komla-Ebri, D., Dambroise, E., Kramer, I., Benoist-Lasselin, C., Kaci, N., Le Gall, C., Martin, L., Busca, P., Barbault, F., Graus-Porta, D., Munnich, A., Kneissel, M., Di Rocco, F., Biosse-Duplan, M., Legeai-Mallet, L., 2016. Tyrosine kinase inhibitor NVP-BGJ398 functionally improves FGFR3-related dwarfism in mouse model. J. Clin. Invest. 126, 1871-1884. https://doi.org/10.1172/JCI83926
Kruszka, P., Addissie, Y.A., Agochukwu, N.B., Doherty, E.S., Muenke, M., 1993. Muenke Syndrome, in: Adam, M.P., Ardinger, H.H., Pagon, R.A., Wallace, S.E., Bean, L.J., Stephens, K., Amemiya, A. (Eds.), GeneReviews®. University of Washington, Seattle, Seattle (WA).
Laurita, J., Koyama, E., Chin, B., Taylor, J.A., Lakin, G.E., Hankenson, K.D., Bartlett, S.P., Nah, H.-D., 2011. The Muenke syndrome mutation (FgfR3P244R) causes cranial base shortening associated with growth plate dysfunction and premature perichondrial ossification in murine basicranial synchondroses. Dev. Dyn. 240, 2584-2596. https://doi.org/10.1002/dvdy.22752
Li, E., You, M., Hristova, K., 2006. FGFR3 dimer stabilization due to a single amino acid pathogenic mutation. J. Mol. Biol. 356, 600-612. https://doi.org/10.1016/j.jmb.2005.11.077
Linnankivi, Tarja, Outi Mäkitie, Leena Valanne, et Sanna Toiviainen-Salo. 2012. « Neuroimaging and Neurological Findings in Patients with Hypochondroplasia and FGFR3 N540K Mutation ». American Journal of Medical Genetics. Part A 158A (12): 3119-25. https://doi.org/10.1002/ajmg.a.35642.
Manikkam, S. A., K. Chetcuti, K. B. Howell, R. Savarirayan, A. M. Fink, et S. A. Mandelstam. 2018. « Temporal Lobe Malformations in Achondroplasia: Expanding the Brain Imaging Phenotype Associated with FGFR3-Related Skeletal Dysplasias ». American Journal of Neuroradiology 39 (2): 380-84. https://doi.org/10.3174/ajnr.A5468.
Maliepaard, M., Mathijssen, I.M.J., Oosterlaan, J., Okkerse, J.M.E., 2014. Intellectual, Behavioral, and Emotional Functioning in Children With Syndromic Craniosynostosis. Pediatrics 133, e1608-e1615. https://doi.org/10.1542/peds.2013-3077
Martínez-Abadías, N., Heuzé, Y., Wang, Y., Jabs, E.W., Aldridge, K., Richtsmeier, J.T., 2011. FGF/FGFR Signaling Coordinates Skull Development by Modulating Magnitude of Morphological Integration: Evidence from Apert Syndrome Mouse Models. PLoS One 6. https://doi.org/10.1371/journal.pone.0026425
Merrill, A.E., Sarukhanov, A., Krejci, P., Idoni, B., Camacho, N., Estrada, K.D., Lyons, K.M., Deixler, H., Robinson, H., Chitayat, D., Curry, C.J., Lachman, R.S., Wilcox, W.R., Krakow, D., 2012. Bent bone dysplasia-FGFR2 type, a distinct skeletal disorder, has deficient canonical FGF signaling. Am. J. Hum. Genet. 90, 550-557. https://doi.org/10.1016/j.ajhg.2012.02.005
Meyers, G.A., Orlow, S.J., Munro, I.R., Przylepa, K.A., Jabs, E.W., 1995. Fibroblast growth factor receptor 3 (FGFR3) transmembrane mutation in Crouzon syndrome with acanthosis nigricans. Nat. Genet. 11, 462-464. https://doi.org/10.1038/ng1295-462
Mir A, Wu T, Orlow SJ, 2013. CUtaneous features of crouzon syndrome with acanthosis nigricans. JAMA Dermatol 149, 737-741. https://doi.org/10.1001/jamadermatol.2013.3019
Moldrich, R.X., Mezzera, C., Holmes, W.M., Goda, S., Brookfield, S.J., Rankin, A.J., Barr, E., Kurniawan, N., Dewar, D., Richards, L.J., López-Bendito, G., Iwata, T., 2011. Fgfr3 regulates development of the caudal telencephalon. Developmental Dynamics 240, 1586-1599. https://doi.org/10.1002/dvdy.22636
Muenke, M., Gripp, K.W., McDonald-McGinn, D.M., Gaudenz, K., Whitaker, L.A., Bartlett, S.P., Markowitz, R.I., Robin, N.H., Nwokoro, N., Mulvihill, J.J., Losken, H.W., Mulliken, J.B., Guttmacher, A.E., Wilroy, R.S., Clarke, L.A., Hollway, G., Adès, L.C., Haan, E.A., Mulley, J.C., Cohen, M.M., Bellus, G.A., Francomano, C.A., Moloney, D.M., Wall, S.A., Wilkie, A.O.M., Zackai, E.H., 1997. A unique point mutation in the fibroblast growth factor receptor 3 gene (FGFR3) defines a new craniosynostosis syndrome. Am J Hum Genet 60, 555-564.
Neben, C.L., Idoni, B., Salva, J.E., Tuzon, C.T., Rice, J.C., Krakow, D., Merrill, A.E., 2014. Bent bone dysplasia syndrome reveals nucleolar activity for FGFR2 in ribosomal DNA transcription. Hum. Mol. Genet. 23, 5659-5671. https://doi.org/10.1093/hmg/ddu282
Neben, C.L., Tuzon, C.T., Mao, X., Lay, F.D., Merrill, A.E., 2017. FGFR2 mutations in bent bone dysplasia syndrome activate nucleolar stress and perturb cell fate determination. Hum. Mol. Genet. 26, 3253-3270. https://doi.org/10.1093/hmg/ddx209
Numakawa, Tadahiro, Haruki Odaka, Naoki Adachi, Shuichi Chiba, Yoshiko Ooshima, Hitomi Matsuno, Shingo Nakajima, et al. 2018. « Basic Fibroblast Growth Factor Increased Glucocorticoid Receptors in Cortical Neurons through MAP Kinase Pathway ».
Okubo, Y., Kitamura, T., Anzai, M., Endo, W., Inui, T., Takezawa, Y., Suzuki-Muromoto, S., Miyabayashi, T., Togashi, N., Oba, H., Saitsu, H., Matsumoto, N., Haginoya, K., 2017. A patient with Muenke syndrome manifesting migrating neonatal seizures. Brain Dev. https://doi.org/10.1016/j.braindev.2017.05.007
Richtsmeier, J.T., Flaherty, K., 2013. Hand in glove: brain and skull in development and dysmorphogenesis. Acta Neuropathol. 125, 469-489. https://doi.org/10.1007/s00401-013-1104-y
Robin, N.H., Falk, M.J., Haldeman-Englert, C.R., 1993. FGFR-Related Craniosynostosis Syndromes, in: Adam, M.P., Ardinger, H.H., Pagon, R.A., Wallace, S.E., Bean, L.J., Stephens, K., Amemiya, A. (Eds.), GeneReviews®. University of Washington, Seattle, Seattle (WA).
Rousseau, F., Bonaventure, J., Legeai-Mallet, L., Pelet, A., Rozet, J.M., Maroteaux, P., Le Merrer, M., Munnich, A., 1994. Mutations in the gene encoding fibroblast growth factor receptor-3 in achondroplasia. Nature 371, 252-254. https://doi.org/10.1038/371252a0
Salmaso, N., Stevens, H.E., McNeill, J., ElSayed, M., Ren, Q., Maragnoli, M.E., Schwartz, M.L., Tomasi, S., Sapolsky, R.M., Duman, R., Vaccarino, F.M., 2016. Fibroblast Growth Factor 2 Modulates Hypothalamic Pituitary Axis Activity and Anxiety Behavior Through Glucocorticoid Receptors. Biological Psychiatry, New Insight Into Depression Therapeutics 80, 479-489. https://doi.org/10.1016/j.biopsych.2016.02.026
Stevens, H.E., Jiang, G.Y., Schwartz, M.L., Vaccarino, F.M., 2012. Learning and memory depend on fibroblast growth factor receptor 2 functioning in hippocampus. Biol Psychiatry 71, 1090-1098. https://doi.org/10.1016/j.biopsych.2012.03.013
Su, N., Xu, X., Li, Cuiling, He, Q., Zhao, L., Li, Can, Chen, S., Luo, F., Yi, L., Du, X., Huang, H., Deng, C., Chen, L., 2010. Generation of Fgfr3 conditional knockout mice. Int. J. Biol. Sci. 6, 327-332.
Tang, Ming-ming, Wen-juan Lin, Yu-qin Pan, et Ying-cong Li. 2018. « Fibroblast Growth Factor 2 Modulates Hippocampal Microglia Activation in a Neuroinflammation Induced Model of Depression ». Frontiers in Cellular Neuroscience 12 (août). https://doi.org/10.3389/fncel.2018.00255.
Thomson, R.E., Kind, P.C., Graham, N.A., Etherson, M.L., Kennedy, J., Fernandes, A.C., Marques, C.S., Hevner, R.F., Iwata, T., 2009. Fgf receptor 3 activation promotes selective growth and expansion of occipitotemporal cortex. Neural Dev 4, 4. https://doi.org/10.1186/1749-8104-4-4
Thomson, R.E., Pellicano, F., Iwata, T., 2007. Fibroblast growth factor receptor 3 kinase domain mutation increases cortical progenitor proliferation via mitogen-activated protein kinase activation. Journal of Neurochemistry 100, 1565-1578. https://doi.org/10.1111/j.1471-4159.2006.04285.x
Twigg et al., 2008. Skeletal Analysis of the Fgfr3P244R Mouse, a Genetic Model for the Muenke Craniosynostosis Syndrome.
Twigg, S.R.F., Wilkie, A.O.M., 2015. A Genetic-Pathophysiological Framework for Craniosynostosis. American Journal of Human Genetics 97, 359. https://doi.org/10.1016/j.ajhg.2015.07.006
Wang, Lin, Xi-Xi Li, Xi Chen, Xiao-Yan Qin, Elissavet Kardami, et Yong Cheng. 2018. « Antidepressant-Like Effects of Low- and High-Molecular Weight FGF-2 on Chronic Unpredictable Mild Stress Mice ». Frontiers in Molecular Neuroscience 11 (octobre). https://doi.org/10.3389/fnmol.2018.00377.
Wilkie, A.O.M., Byren, J.C., Hurst, J.A., Jayamohan, J., Johnson, D., Knight, S.J.L., Lester, T., Richards, P.G., Twigg, S.R.F., Wall, S.A, 2010. Prevalence and complications of single gene and chromosomal disorders in craniosynostosis. Pediatrics 126, e391-e400. https://doi.org/10.1542/peds.2009-3491
Yarnell, C.M.P., Addissie, Y.A., Hadley, D.W., Guillen Sacoto, M.J., Agochukwu, N.B., Hart, R.A., Wiggs, E.A., Platte, P., Paelecke, Y., Collmann, H., Schweitzer, T., Kruszka, P., Muenke, M., 2015. Executive Function and Adaptive Behavior in Muenke Syndrome. J. Pediatr. 167, 428-434. https://doi.org/10.1016/j.jpeds.2015.04.080
Zhou, S., Xie, Y., Tang, J., Huang, J., Huang, Q., Xu, W., Wang, Z., Luo, F., Wang, Q., Chen, H., Du, X., Shen, Y., Chen, D., Chen, L., 2015. FGFR3 Deficiency Causes Multiple Chondroma-like Lesions by Upregulating Hedgehog Signaling. PLoS Genetics 11, undefined-undefined. https://doi.org/10.1371/journal.pgen.1005214

## Claims

1. A therapeutically effective amount of at least one catechin for use in a method of treating a cognitive deficit that is caused by an abnormal overactivation of FGFR3 in the brain in a subject.

2. The therapeutically effective amount of at least one catechin for use according to claim 1 wherein the subject harbours a FGFR3 gain-of-function mutation.

3. The therapeutically effective amount of at least one catechin for use according to claim 2 wherein the FGFR3 gain-of-function mutation is the N540K, K650N, K650Q, M528I, I538V, N540S or N540T mutation.

4. The therapeutically effective amount of at least one catechin for use according to claim 2 wherein the FGFR3 gain-of-function mutation is the A391E mutation.

5. The therapeutically effective amount of at least one catechin for use according to claim 1 wherein the catechin is (+)-catechin.

6. The therapeutically effective amount of at least one catechin for use according to claim 1 wherein the catechin is (-)-catechin.

7. The therapeutically effective amount of at least one catechin for use according to claim 1 wherein the catechin is (+)-epicatechin.

8. The therapeutically effective amount of at least one catechin for use according to claim 1 wherein the catechin is (-)-epicatechin.

9. The therapeutically effective amount of at least one catechin for use according to claim 1 wherein the subject suffers from a skeletal disease that is caused by an abnormal increased activation of FGFR3.

10. The therapeutically effective amount of at least one catechin for use according to claim 9 wherein the skeletal disease is selected from the group consisting of hypochondroplasia (HCH), achondroplasia (ACH), thanatophoric dysplasia (TD), craniosynostosis and dwarfism.

11. The therapeutically effective amount of at least one catechin for use according to claim 10 wherein the skeletal disease is hypochondroplasia (HCH).

12. The therapeutically effective amount of at least one catechin for use according to claim 10 wherein the skeletal disease is achondroplasia (ACH).

13. The therapeutically effective amount of at least one catechin for use according to claim 10 wherein the skeletal disease is craniosynostosis.

14. The therapeutically effective amount of at least one catechin for use according to claim 13 wherein the craniosynostosis is Crouzon syndrome with acanthosis nigricans (CAN).

15. The therapeutically effective amount of at least one catechin for use according to claim 1 wherein the subject is administered with a food composition comprising the therapeutically effective amount of the catechin.

## Patentansprüche

1. Therapeutisch wirksame Menge von mindestens einem Catechin zur Verwendung in einem Verfahren zur Behandlung eines kognitiven Defizits, das durch eine abnormale Überaktivierung von FGFR3 im Gehirn eines Subjekts verursacht ist.

2. Die therapeutisch wirksame Menge von mindestens einem Catechin zur Verwendung gemäß Anspruch 1, wobei das Subjekt eine FGFR3-Gain-of-Function-Mutation aufweist.

3. Die therapeutisch wirksame Menge von mindestens einem Catechin zur Verwendung gemäß Anspruch 2, wobei die FGFR3-Gain-of-Function-Mutation die N540K-, K650N-, K650Q-, M528I-, I538V-, N540S- oder N540T-Mutation ist.

4. Die therapeutisch wirksame Menge von mindestens einem Catechin zur Verwendung gemäß Anspruch 2, wobei die FGFR3-Gain-of-Function-Mutation die A391E-Mutation ist.

5. Die therapeutisch wirksame Menge von mindestens einem Catechin zur Verwendung gemäß Anspruch 1, wobei das Catechin (+ )-Catechin ist.

6. Die therapeutisch wirksame Menge von mindestens einem Catechin zur Verwendung gemäß Anspruch 1, wobei das Catechin (-)-Catechin ist.

7. Die therapeutisch wirksame Menge von mindestens einem Catechin zur Verwendung gemäß Anspruch 1, wobei das Catechin (+)-Epicatechin ist.

8. Die therapeutisch wirksame Menge von mindestens einem Catechin zur Verwendung gemäß Anspruch 1, wobei das Catechin (-)-Epicatechin ist.

9. Die therapeutisch wirksame Menge von mindestens einem Catechin zur Verwendung gemäß Anspruch 1, wobei das Subjekt an einer Skeletterkrankung leidet, die durch eine abnormale erhöhte Aktivierung von FGFR3 verursacht wird.

10. Die therapeutisch wirksame Menge von mindestens einem Catechin zur Verwendung gemäß Anspruch 9, wobei die Skeletterkrankung ausgewählt ist aus der Gruppe bestehend aus Hypochondroplasie (HCH), Achondroplasie (ACH), Thanatophorer Dysplasie (TD), Kraniosynostose und Kleinwuchs.

11. Die therapeutisch wirksame Menge von mindestens einem Catechin zur Verwendung gemäß Anspruch 10, wobei die Skeletterkrankung Hypochondroplasie (HCH) ist.

12. Die therapeutisch wirksame Menge von mindestens einem Catechin zur Verwendung gemäß Anspruch 10, wobei die Skeletterkrankung Achondroplasie (ACH) ist.

13. Die therapeutisch wirksame Menge von mindestens einem Catechin zur Verwendung gemäß Anspruch 10, wobei die Skeletterkrankung eine Kraniosynostose ist.

14. Die therapeutisch wirksame Menge von mindestens einem Catechin zur Verwendung gemäß Anspruch 13, wobei die Kraniosynostose ein Crouzon-Syndrom mit Acanthosis nigricans (CAN) ist.

15. Die therapeutisch wirksame Menge von mindestens einem Catechin zur Verwendung gemäß Anspruch 1, wobei dem Subjekt eine Nahrungsmittelzusammensetzung verabreicht wird, die die therapeutisch wirksame Menge des Catechins umfasst.

## Revendications

1. Quantité thérapeutiquement efficace d'au moins une catéchine destinée à être utilisée dans un procédé de traitement d'un déficit cognitif qui est provoqué par une suractivation anormale de FGFR3 dans le cerveau chez un sujet.

2. Quantité thérapeutiquement efficace d'au moins une catéchine destinée à être utilisée selon la revendication 1, dans laquelle le sujet porte une mutation gain de fonction FGFR3.

3. Quantité thérapeutiquement efficace d'au moins une catéchine destinée à être utilisée selon la revendication 2, dans laquelle la mutation gain de fonction FGFR3 est la mutation N540K, K650N, K650Q, M528I, I528V, N540S ou N540T.

4. Quantité thérapeutiquement efficace d'au moins une catéchine destinée à être utilisée selon la revendication 2, dans laquelle la mutation gain de fonction FGFR3 est la mutation A391E.

5. Quantité thérapeutiquement efficace d'au moins une catéchine destinée à être utilisée selon la revendication 1, dans laquelle la catéchine est de la (+)-catéchine.

6. Quantité thérapeutiquement efficace d'au moins une catéchine destinée à être utilisée selon la revendication 1, dans laquelle la catéchine est de la (-)-catéchine.

7. Quantité thérapeutiquement efficace d'au moins une catéchine destinée à être utilisée selon la revendication 1, dans laquelle la catéchine est de la (+)-épicatéchine.

8. Quantité thérapeutiquement efficace d'au moins une catéchine destinée à être utilisée selon la revendication 1, dans laquelle la catéchine est de la (-)-épicatéchine.

9. Quantité thérapeutiquement efficace d'au moins une catéchine destinée à être utilisée selon la revendication 1, dans laquelle le sujet souffre d'une maladie squelettique qui est provoquée par une activation augmentée anormale de FGFR3.

10. Quantité thérapeutiquement efficace d'au moins une catéchine destinée à être utilisée selon la revendication 9, dans laquelle la maladie squelettique est sélectionnée parmi le groupe constitué de l'hypochondroplasie (HCH), l'achondroplasie (ACH), la dysplasie thanatophore (TD), la craniosynostose et le nanisme.

11. Quantité thérapeutiquement efficace d'au moins une catéchine destinée à être utilisée selon la revendication 10, dans laquelle la maladie squelettique est l'hypochondroplasie (HCH).

12. Quantité thérapeutiquement efficace d'au moins une catéchine destinée à être utilisée selon la revendication 10, dans laquelle la maladie squelettique est l'achondroplasie (ACH).

13. Quantité thérapeutiquement efficace d'au moins une catéchine destinée à être utilisée selon la revendication 10, dans laquelle la maladie squelettique est la craniosynostose.

14. Quantité thérapeutiquement efficace d'au moins une catéchine destinée à être utilisée selon la revendication 13, dans laquelle la craniosynostose est le syndrome de Crouzon avec acanthosis nigricans (CAN).

15. Quantité thérapeutiquement efficace d'au moins une catéchine destinée à être utilisée selon la revendication 1, dans laquelle une composition alimentaire comprenant la quantité thérapeutiquement efficace de la catéchine est administrée au sujet.
